# EUROPÄISCHE PATENTSCHRIFT

(11) **EP 3 223 787 B1**
(45) Veröffentlichungstag und Bekanntmachung des Hinweises auf die Patenterteilung: **11.09.2019**
(21) Anmeldenummer: 15771640.8
(22) Anmeldetag: 02.10.2015
(51) Int. Cl.: A61K 8/49, A61Q 5/06

(54) **MITTEL ZUM FÄRBEN VON KERATINISCHEN FASERN, ENTHALTEND MINDESTENS EINEN DIMEREN, DIKATIONISCHEN AZOFARBSTOFF UND MINDESTENS EIN NICHTIONISCHES TENSID**
AGENTS FOR DYEING KERATIN FIBRES, CONTAINING AT LEAST ONE DIMERIC, DICATIONIC AZO DYE AND AT LEAST ONE NON-IONIC SURFACTANT
AGENTS DE COLORATION DE FIBRES KÉRATINIQUES, CONTENANT AU MOINS UN COLORANT AZOÏQUE DICATIONIQUE DIMÈRE ET AU MOINS UN TENSIOACTIF NON IONIQUE

(30) Priorität: 25.11.2014 DE 102014223938
(43) Veröffentlichungstag der Anmeldung: 04.10.2017
(73) Patentinhaber: Henkel AG & Co. KGaA, 40589 Düsseldorf (DE)
(72) Erfinder: GEBERT-SCHWARZWÄLDER, Antje, 41469 Neuss (DE); MOCH, Melanie, 41542 Dormagen (DE)
(86) Internationale Anmeldenummer: PCT/EP2015/072774
(87) Internationale Veröffentlichungsnummer: WO 2016/082999

(56) Entgegenhaltungen:
- WO-A1-2013/068311
- US-A1- 2001 001 333
- US-B2- 7 407 516

## Beschreibung

Die vorliegende Erfindung betrifft Mittel zum Färben von keratinischen Fasern, insbesondere menschlichen Haaren, die (a) mindestens einen dimeren, dikationischen Azofarbstoff einer speziellen Formel (I) in Kombination mit (b) mindestens einem nichtionischen Tensid enthalten.

Es hat sich herausgestellt, dass durch den Einsatz von speziellen nichtionischen Tensiden (b) die Farbintensität von kationischen Azofarbstoffen, insbesondere von den speziellen Azofarbstoffen der Formel (I), erhöht werden kann.

Für das Färben von keratinischen Fasern kommen im allgemeinen entweder direktziehende Farbstoffe oder Oxidationsfarbstoffe zur Anwendung. Mit Oxidationsfarbstoffen lassen sich zwar intensive Färbungen mit guten Echtheitseigenschaften erzielen, die Entwicklung der Farbe geschieht jedoch im allgemeinen unter dem Einfluss von Oxidationsmitteln wie z. B. H₂O₂, was in einigen Fällen Schädigungen der Faser zur Folge haben kann. Des Weiteren können einige Oxidationsfarbstoffvorprodukte bzw. bestimmte Mischungen von Oxidationsfarbstoffvorprodukten bei Personen mit empfindlicher Haut sensibilisierend wirken. Direktziehende Farbstoffe werden unter schonenderen Bedingungen appliziert. Ihr Nachteil liegt jedoch darin, dass die Färbungen häufig nur über unzureichende Echtheitseigenschaften verfügen.

Abhängig vom gewünschten Farbergebnis setzt der Fachmann direktziehende Farbstoffe unterschiedlicher Farbstoffklassen ein. Die aus dem Stand der Technik bekannten direktziehenden Farbstoffe gehören beispielsweise zur Klasse der Nitrofarbstoffe, der Anthrachinonfarbstoffe, der Azofarbstoffe, der Triarylmethanfarbstoffe oder der Methinfarbstoffe. Alle diese Farbstoffklassen sollten für die Anwendung im Kosmetikbereich ein bestimmtes Anforderungsprofil erfüllen. So sollten direktziehende Farbstoffe ein intensives Färbeergebnis liefern und möglichst gute Echtheitseigenschaften besitzen. Durch Umwelteinflüsse sollte das mit direktziehenden Farbstoffen erhaltene Farbergebnis möglichst wenig beeinflusst werden, d.h. die Farbstoffe sollten beispielsweise eine gute Waschechtheit, Lichtechtheit und Reibechtheit besitzen. Auch chemische Einflüsse, welchen die keratinischen Fasern nach dem Färbeprozess ausgesetzt sein können (wie beispielsweise Dauerwellen), sollten das Farbergebnis möglichst wenig verändern.

Um gleichzeitig mit der Färbung auch eine Aufhellung zu erreichen, sollten die direktziehenden Farbstoffe nach Möglichkeit auch mit den bei Blondierverfahren üblicherweise eingesetzten Oxidationsmitteln (wie z.B. Wasserstoffperoxid und/oder Persulfate) kompatibel sein.

Als Farbstoffklasse mit einem herausragenden Anforderungsprofil haben sich kationische Azofarbstoffe herausgestellt. Azofarbstoffe zeichnen sich generell durch eine hohe Stabilität aus. Darüber hinaus besitzen kationische Azofarbstoffe aufgrund ihrer positiven Ladung eine hohe Affinität zur keratinischen Faser, die abhängig von ihrem Schädigungsgrad mehr oder weniger stark negativ aufgeladenen sein kann.

Sollen keratinische Fasern oxidativ aufgehellt oder blondiert werden, können direktziehende Farbstoffe auch in Kombination mit Oxidationsmitteln eingesetzt werden. Zum Blondieren von Haaren werden in der Regel wässrige Wasserstoffperoxid-Lösungen - entweder allein oder in Kombination mit weiteren, als Bleichaktivatoren wirkenden Oxidationsmitteln wie beispielsweise Persulfatsalzen - auf die Keratinfasern aufgebracht. Um eine ausreichende Blondierwirkung zu erzielen, sind derartige Mittel üblicherweise stark alkalisch eingestellt, der pH-Wert liegt dabei in der Regel zwischen 9 und 10,5. Durch die Einwirkung der Oxidationsmittel werden die Melanine, die natürlichen, farbgebenden Pigmente der Haarfaser, oxidativ zerstört und auf diese Weise eine Entfärbung bzw. Aufhellung der Fasern erreicht. Die Melanine sind im Cortex der Haarfaser lokalisiert und lassen sich in zwei Pigmentklassen einordnen. Eumelanine stellen die erste, bräunlich-schwarzen Pigmentklasse dar, während die rötlichen, schwefelreicheren Pigmente als Pheomelanine bezeichnet werden. Aufgrund der unterschiedlichen Resistenzen der verschiedenen Pigmenttypen gegenüber Oxidationsmitteln werden die Phäo- und Eumelanine jedoch nicht immer gleichmäßig entfärbt. Zudem kann in dunkleren Haaren mit hohem Melaningehalt der Abbau der Melanine nur teilweise oder unvollständig erfolgen, so dass nach der Blondierung ein Restanteil der farbgebenden Pigmente im Haar verbleibt. In diesen Fällen führt der Restgehalt der nach dem oxidativen Prozess im Haar noch vorhandenen Melanine zu einer gelblichen bis rötlichen Nuancenverschiebung. Daher kommt es insbesondere beim Blondieren von dunkleren Haaren zu einer Farbverschiebung in Richtung warmer (rötlicher) Töne.

Üblicherweise sind solche Farbverschiebungen in Richtung warmer Töne beim Anwender unerwünscht. Daher wird dieser Farbverschiebung zumeist durch eine Tönung in der entsprechenden Komplementärfarbe entgegengewirkt. Ziel ist dabei ein silbrig-kühler Eindruck des Bleichergebnisses. Der Fachmann spricht in diesem Zusammenhang von einer Mattierung.

Zur Mattierung von orange-stichigen Blondnuancen können insbesondere blaue, direktziehende Farbstoffe eingesetzt werden. Für die möglichst vollständige Abschwächung des orangen Farbeindrucks ist es hierbei von Vorteil, wenn der blaue Farbstoff selbst keinen Rotanteil in seiner Färbung besitzt. Farbstoffe in reinen Blautönen sind im Vergleich zu violettstichigen Blaufarbstoffen daher besser zur Mattierung eines zu orangen Blondierergebnisses geeignet.

Innerhalb der Gruppe der in Marktprodukten einsetzbaren direktziehenden Blaufarbstoffe gibt es nur sehr wenige Vertreter, welche die Färbung in reinen Blaunuancen erlauben und welche keinen violettstichigen Farbeindruck hinterlassen. Aus dem Stand der Technik sind keine Farbstoffe bekannt, die alle vorgenannten Voraussetzungen in optimaler Weise erfüllen. Es besteht daher weiterhin ein großer Bedarf an stabilen Farbstoffen, welche die keratinischen Fasern in reinen Blautönen färben, und welche ein intensives Farbeergebnis mit herausragenden Echtheitseigenschaften liefern.

US 7407516 B1 beschreibt Färbemittel für keratinische Fasern, die spezielle dikationische Diazo-Farbstoffe enthalten. In US 2001/0001333 A1 wird ein Verfahren zum Färben von Keratinfasern adressiert, wobei ein Mittel mit bestimmten Farbstoffen auf den Haaren appliziert wird. In WO 2013/068311 A1 werden bestimmte anionische Azofarbstoffe zum Färben von Keratinfasern eingesetzt. Aus dem Stand der Technik hinlänglich bekannte monomere kationische Azofarbstoffe sind beispielsweise die Vertreter Basic Orange 31 (Alternativname: 2-[(4-Aminophenyl)azo]-1,3-dimethyl-1H-imidazolium chloride, CAS-Nr. 97404-02-9) und Basic Red 51 (Alternativname: 2-[((4-Dimethylamino)phenyl)azo]-1,3-dimethyl-1H-imidazolium chlorid, CAS-Nr. 77061-58-6).

Beide Farbstoffe färben keratinischen Fasern mit herausragender Farbintensität in orange bis roten Nuancenbereich. Es besteht weiterhin auch noch Bedarf an direktziehenden Blaufarbstoffen, welche mit diesen beiden Farbstoffen in optimaler Weise kompatibel sind.

Es war daher die Aufgabe der vorliegenden Erfindung, auf direktziehenden Farbstoffen basierende Färbemittel zu finden, welche die üblichen, an diese Mittel gestellten Echtheitsanforderungen erfüllen und welche keratinische Fasern in einem reinen Blauton ohne Rotanteil zu färben vermögen. Die mit diesen Mitteln erzielbaren Färbungen sollten eine besonders hohe Farbintensität besitzen.

Darüber hinaus sollen die in den Mitteln enthaltenen Blaufarbstoffe auch besonders gut mit den kationischen Azofarbstoffen Basic Orange 31 und Basic Red 51 kompatibel sein. Überraschenderweise konnte nun gefunden werden, dass diese Aufgabe in hervorragendem Maße erfüllt wird, wenn Farbstoffe der nachfolgend beschriebenen Formel (I) in Kombination mit mindestens einem nichtionischen Tensid in Mitteln zum Färben von keratinischen Fasern eingesetzt werden.

Ein erster Gegenstand der vorliegenden Erfindung ist ein Mittel zum Färben von keratinischen Fasern, insbesondere menschlichen Haaren, enthaltend in einem kosmetischen Träger
(a) mindestens einen direktziehenden Farbstoff der Formel (I), 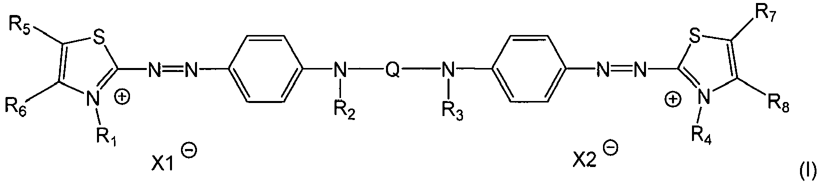 wobei
   - R1,R4: unabhängig voneinander für eine C₁-C₆-Alkylgruppe stehen,
   - R2, R3: unabhängig voneinander für ein Wasserstoffatom oder eine C₁-C₆-Alkylgruppe stehen,
   - R5, R6, R7, R8: jeweils für ein Wasserstoffatom stehen,
   - X1, X2: unabhängig voneinander für ein physiologisch verträgliches Anion, bevorzugt aus der Gruppe Chlorid, Bromid, lodid, Methylsulfat, Methylsulfonat, p-Toluolsulfonat, Acetat, Hydrogensulfat, ½ Sulfat oder ½ Tetrachlorozinkat, stehen,
   - Q: für eine Gruppierung der Formel (II) steht,

   *-(CH₂)n-* (II)
   - n: für eine ganze Zahl von 3 bis 6 steht,
   und
(b) mindestens ein nichtionisches Tensid.

Unter keratinischen Fasern, keratinhaltigen Fasern oder Keratinfasern sind Pelze, Wolle, Federn und insbesondere menschliche Haare zu verstehen. Obwohl die erfindungsgemäßen Mittel in erster Linie zum Aufhellen von Keratinfasern geeignet sind, steht prinzipiell einer Verwendung auch auf anderen Gebieten nichts entgegen.

Der erfindungsgemäß verwendete Begriff "Färben von Keratinfasern" umfasst jedwede Form der Farbveränderung der Fasern. Umfasst sind insbesondere die unter den Begriffen Tönung, Blondierung, Mattierung, oxidativer Färbung, semipermanenter Färbung, permanenter Färbung sowie temporärer Färbung umfassten Farbveränderungen. Explizit mit umfasst sind erfindungsgemäß Farbveränderungen, die ein helleres Farbergebnis im Vergleich zur Ausgangsfarbe aufweisen, wie beispielsweise färbende Blondierungen. Unter dem Begriff "Mattierung von Keratinfasern" wird das Entgegenwirken von unerwünschten Nuancenverschiebungen verstanden, welche bei oxidativen Farbveränderungen von Keratinfasern, insbesondere bei Blondierungen oder bei Bleichprozessen, auftreten. Ziel ist der Mattierung ist die Abschwächung des durch unvollständige Blondierung hervorgerufenen orangen bis rötlichen Farbeindrucks und das Erzeugen einer silbrig-kühlen Farbwahrnehmung nach dem Blondierprozess. Die bei der Mattierung eingesetzten Wirkstoffe können in Form eines Nachbehandlungsschrittes nach dem Blondieren bzw. Bleichen der Keratinsfasern appliziert werden. Es ist aber ebenso möglich, die für die Mattierung eingesetzten Wirkstoffe im Rahmen eines einstufigen Verfahrens zusammen mit dem Blondiermittel bzw. dem Bleichmittel auf die Keratinfasern aufzutragen. Als für die Mattierung geeignete Wirkstoffe können direktziehende Farbstoffe - entweder allein oder im Farbstoffgemisch - mit den geeigneten farblichen Eigenschaften verwendet werden. Darüber hinaus ist es ebenfalls möglich, für die Mattierung direktziehende Farbstoffe in Kombination mit Oxidationsfarbstoffvorprodukten (Entwicklern und Kupplern) einzusetzen.

Die erfindungsgemäßen Mittel enthalten die direktziehenden Farbstoffe der Formel (I) in einem kosmetischen Träger. Dieser kosmetische Träger ist bevorzugt wässrig, alkoholisch oder wässrigalkoholisch. Zum Zwecke der Haarbehandlung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch möglich, zur Lagerung eine pulverförmige oder auch tablettenförmige Formulierung bereitzustellen. Diese wird dann vor der Anwendung in einem wässrigen Lösungsmittel oder mit organischen Lösungsmitteln bzw. mit Gemischen aus Wasser und organischen Lösungsmitteln unter Erhalt der Anwendungsmischung vermengt. Ein wässriger Träger enthält im Sinne der Erfindung mindestens 40 Gew.-%, insbesondere mindestens 50 Gew.-% Wasser. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Bevorzugte erfindungsgemäße Mittel sind dadurch gekennzeichnet, dass sie zusätzlich ein nichtwässriges Lösungsmittel enthalten, wobei bevorzugte erfindungsgemäße Mittel das Lösungsmittel in einer Konzentration von 0,1 bis 30 Gew.-%, bevorzugt in einer Konzentration von 1 bis 20 Gew.-%, ganz besonders bevorzugt in einer Konzentration von 2 bis 10 Gew.-%, jeweils bezogen auf das Mittel, enthalten.

Als ersten wesentlichen Inhaltsstoff (a) enthalten die erfindungsgemäßen Mittel mindestens einen direktziehenden Farbstoff der Formel (I).

Die Substituenten R1 bis R8 der Verbindungen der Formel (I) sind nachstehend beispielhaft erläutert: Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine C₂-C₆-Alkenylgruppe sind Vinyl, Allyl, But-2-enyl, But-3-enyl sowie Isobutenyl, bevorzugte C₂-C₆-Alkenylreste sind Vinyl und Allyl. Bevorzugte Beispiele für eine Hydroxy-C₁-C₆-alkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; eine 2-Hydroxyethylgruppe ist besonders bevorzugt. Bevorzugte Beispiele für Cyano-C₁-C₆-alkylgruppen sind die Cyanomethylgruppe, die 2-Cyanoethylgruppe und die 3-Cyanopropylgruppe. Erfindungsgemäß bevorzugte Halogen-C₁-C₆-alkylgruppen sind die Chlormethylgruppe, die Brommethylgruppe, die Fluormethylgruppe, die 2-Chlorethylgruppe, die 2-Bromethylgruppe, die 2-Fluormethylgruppe, die 2-Chlorpropylgruppe, die 2-Brompropylgruppe, die 2-Fluropropylgruppe, die 3-Chlorpropylgruppe, die 3-Brompropylgruppe und die 3-Fluorpropylgruppe. Bevorzugte Beispiele von Aryl-C₁-C₆-alkylgruppen sind Benzyl, 1-Phenethyl und 2-Phenylethyl. Beispielhaft für Heteroaryl-C₁-C₆-alkylgruppen können die Imidazol-1-ylmethylgruppe, die Imidazol-2-ylmethylgruppe, die Imidazol-4-ylmethylgruppe, die Pyridin-2-ylgruppe, die Pyridin-3-ylgruppe und die Pyridin-4-ylmethylgruppe genannt werden. Erfindungsgemäß bevorzugte Arylgruppen sind die Phenylgruppe und die Naphthylgruppe. Beispiele für eine Heteroarylgruppe sind die Pyridin-2-ylgruppe, die Pyridin-3-ylgruppe, die Pyridin-4-ylgruppe, die Imidazol-1-ylgruppe, die Imidazol-2-ylgruppe und die Imidazol-4-ylgruppe. Halogenatome sind ausgewählt aus der Gruppe Chlor, Brom, Fluor und/oder Jod, hierbei sind Chlor und Brom besonders bevorzugt. Beispielhaft für eine C1-C6-Alkoxygruppe können die Methoxy-, die Ethoxy- und die Propoxygruppe genannt werden.

Die Verbindungen der allgemeinen Formel (I) tragen die Reste R1 und R4; hierbei können R1 und R4 gleich oder verschieden sein. Bevorzugt sind die Reste R1 und R4 gleich. R1 und R4 stehen unabhängig voneinander für eine C₁-C₆-Alkylgruppe, insbesondere für eine Methylgruppe oder für eine Ethylgruppe. Weiterhin tragen die Verbindungen der allgemeinen Formel (I) die Reste R2 und R3; hierbei können R2 und R3 gleich oder verschieden sein. Bevorzugt sind die Reste R2 und R3 gleich.

R2, und R3 stehen unabhängig voneinander für ein Wasserstoffatom oder eine C₁-C₆-Alkylgruppe. Ganz besonders bevorzugt stehen R2 und R3 unabhängig voneinander für ein Wasserstoffatom, eine Methylgruppe oder eine Ethylgruppe.

Die Reste R5, R6, R7, und R8 stehen alle für ein Wasserstoffatom.

In einer ganz besonders bevorzugten Ausführungsform ist ein erfindungsgemäßes Mittel dadurch gekennzeichnet, dass es (a) mindestens einen direktziehenden Farbstoff der allgemeinen Formel (I) enthält, in der
- R1, R4: unabhängig voneinander für eine Methylgruppe oder für eine Ethylgruppe stehen,
- R2, R3: unabhängig voneinander für ein Wasserstoffatom, eine Methylgruppe oder für eine Ethylgruppe stehen und
- R5, R6, R7, R8: jeweils für ein Wasserstoffatom stehen.

Bei den erfindungsgemäßen Farbstoffen der Formel (I) handelt es sich um dimere Azofarbstoffe, die zweifach positiv geladen sind. Die beiden positiven Ladungen werden durch die anionischen Gegenionen X1 und X2 neutralisiert. Hierbei ist der dikationische organische Teil für die Blaufärbung der Keratinfasern verantwortlich. Die Gegenionen X1 und X2 dienen lediglich der Wahrung der Elektroneutralität, so dass die genaue Natur der Gegenionen X1 und X2 für die Erzielung des gewünschten Farbergebnisses keine wesentliche Rolle spielt. Da der Farbstoff in einem kosmetischen Mittel eingesetzt wird, müssen die Gegenionen X1 und X2 physiologisch verträglich sein. Physiologisch verträglich bedeutet in diesem Zusammenhang zum Einsatz im kosmetischen Mittel (d.h. zur Anwendung auf dem menschlichen Haar und der menschlichen Haut) geeignet. Bei X1 und X2 handelt es sich um physiologisch verträgliche Anionen, bevorzugt aus der Gruppe Chlorid, Bromid, lodid, Methylsulfat, Methylsulfonat, p-Toluolsulfonat, Acetat, Hydrogensulfat, ½ Sulfat und Tetrachlorozinkat. Unter Chlorid wird ein Anion Cl⁻ verstanden. Unter Bromid wird ein Anion Br verstanden. Unter lodid wird ein Anion J⁻ verstanden. Unter Methylsulfat wird ein Anion H₃COSO₄⁻ verstanden.

Unter Methylsulfonat wird ein Anion H₃CSO₃⁻ verstanden. Unter p-Toluolsulfonat wird ein Anion H₃C(C₆H₄)SO₃⁻ verstanden. Unter Acetat wird ein Anion H₃CCOO⁻ verstanden. Unter Hydrogensulfat wird ein Anion HSO₄⁻ verstanden.

Unter ½ Sulfat wird ein halbes Äquivalent des doppelt negativ geladenen Anions SO₄²⁻ verstanden. Unter ½ Tetrachlorozinkat wird ein halbes Äquivalent des doppelt negativ geladenen Anions ZnCl₄²⁻ verstanden. Bei Sulfat und Tetrachlorozinkat ist es demzufolge ebenfalls möglich und auch erfindungsgemäß, wenn der dikationische Farbstoff der Formel (I) durch ein SO₄²⁻ Ion bzw. durch ein ZnCl₄²⁻ Ion neutralisiert wird.

Bei der Gruppierung Q handelt es sich um eine Gruppierung, welche die beiden einfach positiv geladenen Chromophore des Farbstoffs zum dikationischen Dimer verknüpft. Q steht für eine Gruppierung der Formel (II)

*-(CH₂)n-* (II)

wobei
- n: für eine ganze Zahl von 3 bis 6 steht,

Die beiden mit einem Stern markierten Position stellen hierbei jeweils die Verknüpfungspositionen zu den beiden N-Atomen der Formel (I) dar.

Überraschenderweise hat es sich für die Erzielung eines intensiven Farbergebnisses als grundsätzlich wichtig und erfindungswesentlich herausgestellt, dass die verbindende Gruppierung Q, welche die beiden Azo-Chromophore miteinander verknüpft, eine Kettenlänge von mindestens 3 Atomen aufweist. Aus diesem Grund steht n in der Formel (II) für eine ganze Zahl von mindestens 3. Die verbindende Gruppierung Q der Formel (II) umfasst demnach mindestens 3 C-Atome (d.h. hierbei handelt es sich um eine Gruppierung mit der Mindestlänge von -CH₂-CH₂-CH₂-).

Im Rahmen von Vergleichsversuchen hat sich herausgestellt, dass dimere Azofarbstoffe des prinzipiellen Typs der Formel (I), die jedoch eine nicht erfindungsgemäße Linker-Gruppe Q mit einer Länge von nur 2 C-Atomen besitzen, ein extrem schlechtes Farbaufzugsvermögen auf die Keratinfasern besitzen.

Während mit den erfindungsgemäßen Farbstoffen der Formel (I) intensive Färbungen mit tief dunkelblauem Farbton erzielt werden konnten, führten Färbungen mit analogen dimeren Farbstoffen, die über eine kürzere Gruppierung Q mit einer Kettenlänge von nur 2 C-Atomen verknüpft sind, zu praktisch überhaupt keinem Farbaufzug auf den keratinischen Fasern.

Ohne auf eine Theorie beschränkt zu sein, könnte möglicherweise eine mit der kurzen Linker-Kette Q verbundene starre Geometrie und hierdurch bedingt eine ungünstige räumliche Konformation des Farbstoffes die Diffusion der kurzkettigen dimeren Farbstoffe in die keratinische Faser hinein verschlechtern.

Ein erfindungsgemäßes Mittel ist dadurch gekennzeichnet, dass es (a) mindestens einen direktziehenden Farbstoff der allgemeinen Formel (I) enthält, in der Q für eine Gruppierung der Formel (II) steht,

*-(CH₂)n-* (II)

und
- n: für eine ganze Zahl von 3 bis 6 steht.

In einer explizit ganz besonders bevorzugten Ausführungsform ist ein erfindungsgemäßes Mittel dadurch gekennzeichnet, dass es (a) mindestens einen Farbstoff der allgemeinen Formel (I) enthält, in der
- Q: für eine Gruppierung der Formel (II) steht,

*-(CH₂)n-* (II)
und
- n: für die Zahl 3 steht.

In einer weiteren bevorzugten Ausführungsform ist ein Mittel zum Färben von keratinischen Fasern dadurch gekennzeichnet, dass es mindestens eine Verbindung der allgemeinen Formel (I) enthält, die ausgewählt ist aus
- Salzen des 3-Methyl-2-[2-(4-{[3-({4-[2-(3-methyl-1,3-thiazol-3-ium-2-yl)diazen-1-yl]phenyl}amino)propyl]amino}phenyl)diazen-1-yl]-1,3-thiazol-3-iums 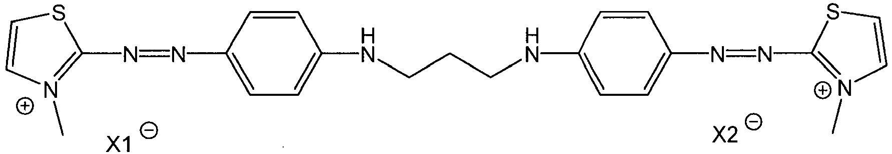
- Salzen des 3-Methyl-2-[2-(4-{[4-({4-[2-(3-methyl-1,3-thiazol-3-ium-2-yl)diazen-1-yl]phenyl}amino)butyl]amino}phenyl)diazen-1-yl]-1,3-thiazol-3-iums 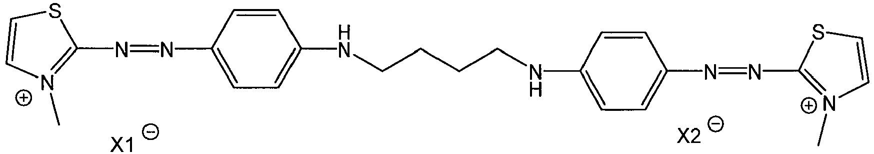
- Salzen des 3-Methyl-2-[2-(4-{[5-({4-[2-(3-methyl-1,3-thiazol-3-ium-2-yl)diazen-1-yl]phenyl}amino)pentyl]amino}phenyl)diazen-1-yl]-1,3-thiazol-3-iums 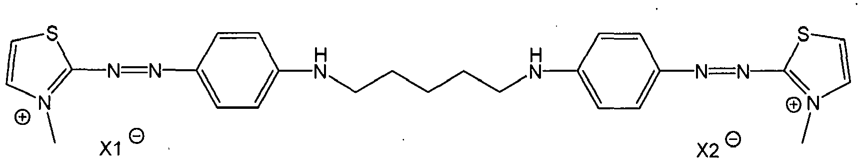
- Salzen des 3-Methyl-2-(2-{4-[methyl({3-[methyl({4-[2-(3-methyl-1,3-thiazol-3-ium-2-yl)diazen-1-yl]phenyl})amino]propyl})amino]phenyl}diazen-1-yl)-1,3-thiazol-3-iums 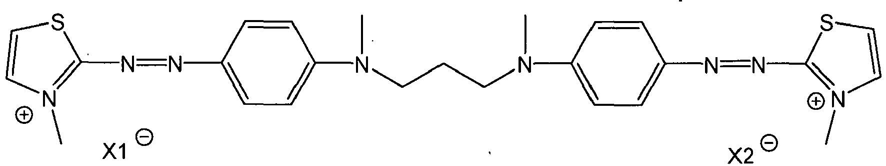
- Salzen des 3-Methyl-2-(2-{4-[methyl({4-[methyl({4-[2-(3-methyl-1,3-thiazol-3-ium-2-yl)diazen-1-yl]phenyl})amino]butyl})amino]phenyl}diazen-1-yl)-1,3-thiazol-3-iums 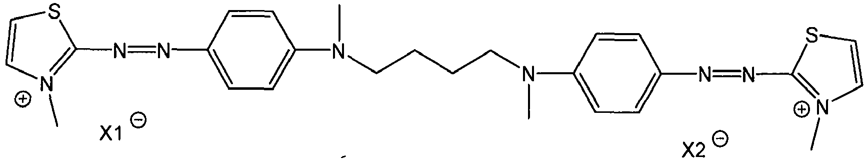
- Salzen des 3-Methyl-2-(2-{4-[methyl({5-[methyl({4-[2-(3-methyl-1,3-thiazol-3-ium-2-yl)diazen-1-yl]phenyl})amino]pentyl})amino]phenyl}diazen-1-yl)-1,3-thiazol-3-iums 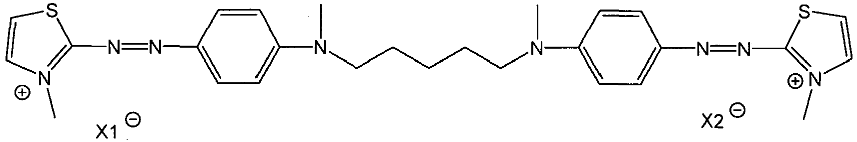
- Salzen des 2-(2-{4-[Ethyl({3-[ethyl({4-[2-(3-methyl-1,3-thiazol-3-ium-2-yl)diazen-1-yl]phenyl})amino]propyl})amino]phenyl}diazen-1-yl)-3-methyl-1,3-thiazol-3-iums 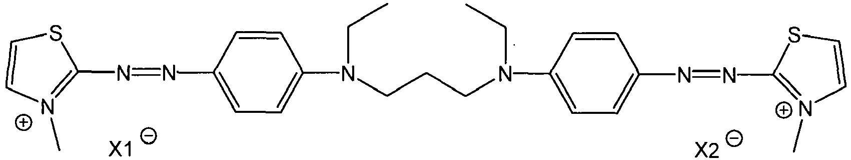
- Salzen des 2-(2-{4-[Ethyl({4-[ethyl({4-[2-(3-methyl-1,3-thiazol-3-ium-2-yl)diazen-1-yl]phenyl})amino]butyl})amino]phenyl}diazen-1-yl)-3-methyl-1,3-thiazol-3-iums 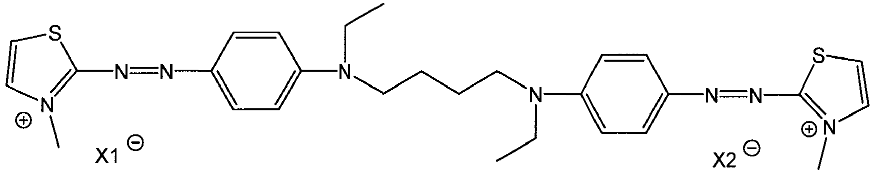
- Salzen des 2-(2-{4-[Ethyl({5-[ethyl({4-[2-(3-methyl-1,3-thiazol-3-ium-2-yl)diazen-1-yl]phenyl})amino]pentyl})amino]phenyl}diazen-1-yl)-3-methyl-1,3-thiazol-3-iums 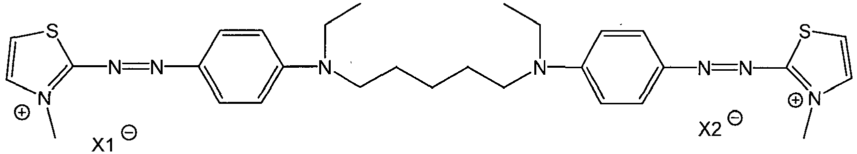
- Salzen des 3-Ethyl-2-[2-(4-{[3-({4-[2-(3-ethyl-1,3-thiazol-3-ium-2-yl)diazen-1-yl]phenyl}amino)propyl]amino}phenyl)diazen-1-yl]-1,3-thiazol-3-iums 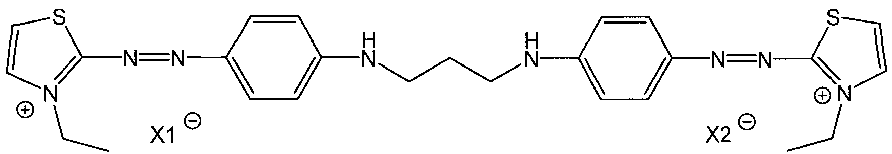
- Salzen des 3-Ethyl-2-[2-(4-{[4-({4-[2-(3-ethyl-1,3-thiazol-3-ium-2-yl)diazen-1-yl]phenyl}amino)butyl]amino}phenyl)diazen-1-yl]-1,3-thiazol-3-iums 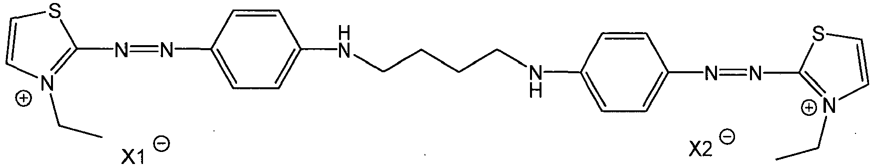
- Salzen des 3-Ethyl-2-[2-(4-{[5-({4-[2-(3-ethyl-1,3-thiazol-3-ium-2-yl)diazen-1-yl]phenyl}amino)pentyl]amino}phenyl)diazen-1-yl]-1,3-thiazol-3-iums 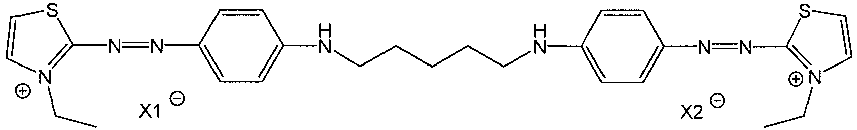
- Salzen des 3-Ethyl-2-[2-(4-{[3-({4-[2-(3-ethyl-1,3-thiazol-3-ium-2-yl)diazen-1-yl]phenyl}(methyl)amino)propyl](methyl)amino}phenyl)diazen-1-yl]-1,3-thiazol-3-iums 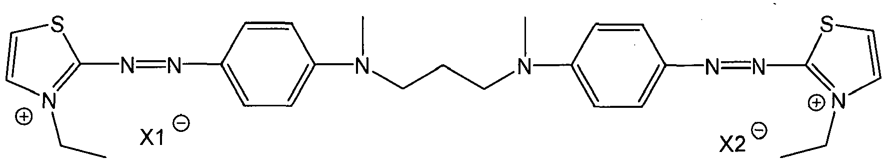
- Salzen des 3-ethyl-2-[2-(4-{[4-({4-[2-(3-ethyl-1,3-thiazol-3-ium-2-yl)diazen-1-yl]phenyl}(methyl)amino)butyl](methyl)amino}phenyl)diazen-1-yl]-1,3-thiazol-3-iums 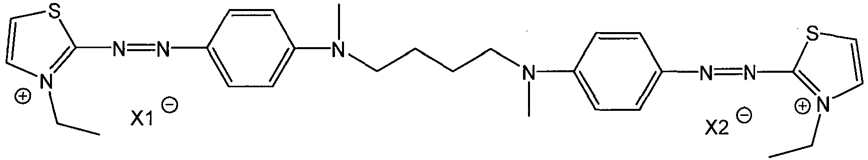
- Salzen des 3-Ethyl-2-[2-(4-{[5-({4-[2-(3-ethyl-1,3-thiazol-3-ium-2-yl)diazen-1-yl]phenyl}(methyl)amino)pentyl](methyl)amino}phenyl)diazen-1-yl]-1,3-thiazol-3-iums 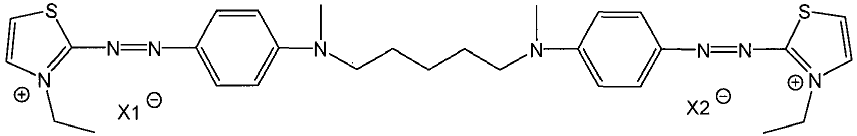
- Salzen des 3-Ethyl-2-(2-{4-[ethyl({3-[ethyl({4-[2-(3-ethyl-1,3-thiazol-3-ium-2-yl)diazen-1-yl]phenyl})amino]propyl})amino]phenyl}diazen-1-yl)-1,3-thiazol-3-iums 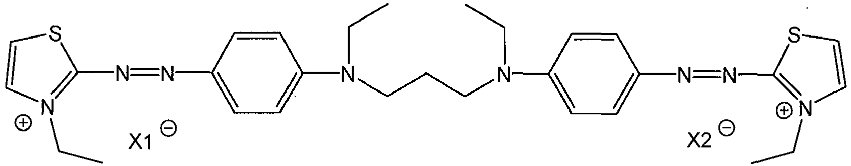
- Salzen des 3-Ethyl-2-(2-{4-[ethyl({4-[ethyl({4-[2-(3-ethyl-1,3-thiazol-3-ium-2-yl)diazen-1-yl]phenyl})amino]butyl})amino]phenyl}diazen-1-yl)-1,3-thiazol-3-iums 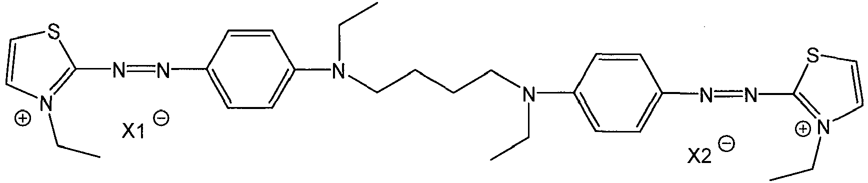
- Salzen des 3-Ethyl-2-(2-{4-[ethyl({5-[ethyl({4-[2-(3-ethyl-1,3-thiazol-3-ium-2-yl)diazen-1-yl]phenyl})amino]pentyl})amino]phenyl}diazen-1-yl)-1,3-thiazol-3-iums

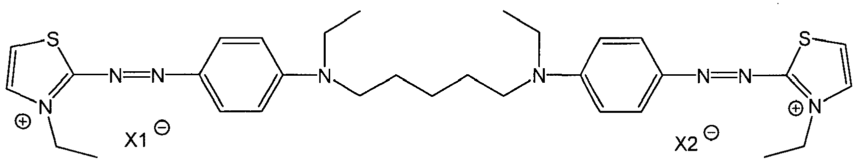

Bei den vorgenannten Verbindungen handelt es sich um dikationische dimere Farbstoffe, wobei das organische Dikation durch die beiden Anionen X1- und X2- neutralisiert wird. Bei den Anionen X1- und X2 kann es sich jeweils um ein physiologisch veträgliches Anion, bevorzugt aus der Gruppe aus Chlorid, Bromid, lodid, Methylsulfat, Methylsulfonat, p-Toluolsulfonat, Acetat, Hydrogensulfat, ½ Sulfat oder ½ Tetrachlorozinkat handeln.

In einer besonders bevorzugten Ausführungsform ist ein erfindungsgemäßes Mittel zum Färben von keratinischen Fasern dadurch gekennzeichnet, dass es als Farbstoff der Formel (a) mindestens eine Verbindung enthält, die ausgewählt ist aus der Gruppe aus
- 3-Methyl-2-[2-(4-{[3-({4-[2-(3-methyl-1,3-thiazol-3-ium-2-yl)diazen-1-yl]phenyl}amino)propyl]amino}phenyl)diazen-1-yl]-1,3-thiazol-3-ium Dichlorid
- 3-Methyl-2-[2-(4-{[3-({4-[2-(3-methyl-1,3-thiazol-3-ium-2-yl)diazen-1-yl]phenyl}amino)propyl]amino}phenyl)diazen-1-yl]-1,3-thiazol-3-ium Dibromid
- 3-Methyl-2-[2-(4-{[3-({4-[2-(3-methyl-1,3-thiazol-3-ium-2-yl)diazen-1-yl]phenyl}amino)propyl]amino}phenyl)diazen-1-yl]-1,3-thiazol-3-ium Sulfat
- 3-Methyl-2-[2-(4-{[3-({4-[2-(3-methyl-1,3-thiazol-3-ium-2-yl)diazen-1-yl]phenyl}amino)propyl]amino}phenyl)diazen-1-yl]-1,3-thiazol-3-ium Di(toluolsulfonat)
- 3-Methyl-2-[2-(4-{[3-({4-[2-(3-methyl-1,3-thiazol-3-ium-2-yl)diazen-1-yl]phenyl}amino)propyl]amino}phenyl)diazen-1-yl]-1,3-thiazol-3-ium Di(methylsulfat)
- 3-Methyl-2-[2-(4-{[3-({4-[2-(3-methyl-1,3-thiazol-3-ium-2-yl)diazen-1-yl]phenyl}amino)propyl]amino}phenyl)diazen-1-yl]-1,3-thiazol-3-ium Tetrachlorozinkat
- 3-Methyl-2-[2-(4-{[4-({4-[2-(3-methyl-1,3-thiazol-3-ium-2-yl)diazen-1-yl]phenyl}amino)butyl]amino}phenyl)diazen-1-yl]-1,3-thiazol-3-ium Dichlorid
- 3-Methyl-2-[2-(4-{[4-({4-[2-(3-methyl-1,3-thiazol-3-ium-2-yl)diazen-1-yl]phenyl}amino)butyl]amino}phenyl)diazen-1-yl]-1,3-thiazol-3-ium Dibromid
- 3-Methyl-2-[2-(4-{[4-({4-[2-(3-methyl-1,3-thiazol-3-ium-2-yl)diazen-1-yl]phenyl}amino)butyl]amino}phenyl)diazen-1-yl]-1,3-thiazol-3-ium Sulfat
- 3-Methyl-2-[2-(4-{[4-({4-[2-(3-methyl-1,3-thiazol-3-ium-2-yl)diazen-1-yl]phenyl}amino)butyl]amino}phenyl)diazen-1-yl]-1,3-thiazol-3-ium Di(toluolsulfonat)
- 3-Methyl-2-[2-(4-{[4-({4-[2-(3-methyl-1,3-thiazol-3-ium-2-yl)diazen-1-yl]phenyl}amino)butyl]amino}phenyl)diazen-1-yl]-1,3-thiazol-3-ium Di(methylsulfat)
- 3-Methyl-2-[2-(4-{[4-({4-[2-(3-methyl-1,3-thiazol-3-ium-2-yl)diazen-1-yl]phenyl}amino)butyl]amino}phenyl)diazen-1-yl]-1,3-thiazol-3-ium Tetrachlorozinkat
- 3-Methyl-2-[2-(4-{[5-({4-[2-(3-methyl-1,3-thiazol-3-ium-2-yl)diazen-1-yl]phenyl}amino)pentyl]amino}phenyl)diazen-1-yl]-1,3-thiazol-3-ium Dichlorid
- 3-Methyl-2-[2-(4-{[5-({4-[2-(3-methyl-1,3-thiazol-3-ium-2-yl)diazen-1-yl]phenyl}amino)pentyl]amino}phenyl)diazen-1-yl]-1,3-thiazol-3-ium Dibromid
- 3-Methyl-2-[2-(4-{[5-({4-[2-(3-methyl-1,3-thiazol-3-ium-2-yl)diazen-1-yl]phenyl}amino)pentyl]amino}phenyl)diazen-1-yl]-1,3-thiazol-3-ium Sulfat
- 3-Methyl-2-[2-(4-{[5-({4-[2-(3-methyl-1,3-thiazol-3-ium-2-yl)diazen-1-yl]phenyl}amino)pentyl]amino}phenyl)diazen-1-yl]-1,3-thiazol-3-ium Di(toluolsulfonat)
- 3-Methyl-2-[2-(4-{[5-({4-[2-(3-methyl-1,3-thiazol-3-ium-2-yl)diazen-1-yl]phenyl}amino)pentyl]amino}phenyl)diazen-1-yl]-1,3-thiazol-3-ium (Di(methylsulfat)
- 3-Methyl-2-[2-(4-{[5-({4-[2-(3-methyl-1,3-thiazol-3-ium-2-yl)diazen-1-yl]phenyl}amino)pentyl]amino}phenyl)diazen-1-yl]-1,3-thiazol-3-ium Tetrachlorozinkat
- 3-Methyl-2-(2-{4-[methyl({3-[methyl({4-[2-(3-methyl-1,3-thiazol-3-ium-2-yl)diazen-1-yl]phenyl})amino]propyl})amino]phenyl}diazen-1-yl)-1,3-thiazol-3-ium Dichlorid
- 3-Methyl-2-(2-{4-[methyl({3-[methyl({4-[2-(3-methyl-1,3-thiazol-3-ium-2-yl)diazen-1-yl]phenyl})amino]propyl})amino]phenyl}diazen-1-yl)-1,3-thiazol-3-ium Dibromid
- 3-Methyl-2-(2-{4-[methyl({3-[methyl({4-[2-(3-methyl-1,3-thiazol-3-ium-2-yl)diazen-1-yl)phenyl})amino]propyl})amino]phenyl}diazen-1-yl)-1,3-thiazol-3-ium Sulfat
- 3-Methyl-2-(2-{4-[methyl({3-[methyl({4-[2-(3-methyl-1,3-thiazol-3-ium-2-yl)diazen-1-yl]phenyl})amino]propyl})amino]phenyl}diazen-1-yl)-1,3-thiazol-3-ium Di(toluolsulfonat)
- 3-Methyl-2-(2-{4-[methyl({3-[methyl({4-[2-(3-methyl-1,3-thiazol-3-ium-2-yl)diazen-1-yl]phenyl})amino]propyl})amino]phenyl}diazen-1-yl)-1,3-thiazol-3-ium Di(methylsulfat)
- 3-Methyl-2-(2-{4-[methyl({3-[methyl({4-[2-(3-methyl-1,3-thiazol-3-ium-2-yl)diazen-1-yl]phenyl})amino]propyl})amino]phenyl}diazen-1-yl)-1,3-thiazol-3-ium Tetrachlorozinkat
- 3-Methyl-2-(2-{4-[methyl({4-[methyl({4-[2-(3-methyl-1,3-thiazol-3-ium-2-yl)diazen-1-yl]phenyl})amino]butyl})amino]phenyl}diazen-1-yl)-1,3-thiazol-3-ium Dichlorid
- 3-Methyl-2-(2-{4-[methyl({4-[methyl({4-[2-(3-methyl-1,3-thiazol-3-ium-2-yl)diazen-1-yl]phenyl})amino]butyl})amino]phenyl}diazen-1-yl)-1,3-thiazol-3-ium Dibromid
- 3-Methyl-2-(2-{4-[methyl({4-[methyl({4-[2-(3-methyl-1,3-thiazol-3-ium-2-yl)diazen-1-yl]phenyl})amino]butyl})amino]phenyl}diazen-1-yl)-1,3-thiazol-3-ium Sulfat
- 3-Methyl-2-(2-{4-[methyl({4-[methyl({4-[2-(3-methyl-1,3-thiazol-3-ium-2-yl)diazen-1-yl]phenyl})amino]butyl})amino]phenyl}diazen-1-yl)-1,3-thiazol-3-ium Di(toluolsulfonat)
- 3-Methyl-2-(2-{4-[methyl({4-[methyl({4-[2-(3-methyl-1,3-thiazol-3-ium-2-yl)diazen-1-yl]phenyl})amino]butyl})amino]phenyl}diazen-1-yl)-1,3-thiazol-3-ium Di(methylsulfat)
- 3-Methyl-2-(2-{4-[methyl({4-[methyl({4-[2-(3-methyl-1,3-thiazol-3-ium-2-yl)diazen-1-yl]phenyl})amino]butyl})amino]phenyl}diazen-1-yl)-1,3-thiazol-3-ium Tetrachlorozinkat
- 3-Methyl-2-(2-{4-[methyl({5-[methyl({4-[2-(3-methyl-1,3-thiazol-3-ium-2-yl)diazen-1-yl]phenyl})amino]pentyl})amino]phenyl}diazen-1-yl)-1,3-thiazol-3-ium Dichlorid
- 3-Methyl-2-(2-{4-[methyl({5-[methyl({4-[2-(3-methyl-1,3-thiazol-3-ium-2-yl)diazen-1-yl]phenyl})amino]pentyl})amino]phenyl}diazen-1-yl)-1,3-thiazol-3-ium Dibromid
- 3-Methyl-2-(2-{4-[methyl({5-[methyl({4-[2-(3-methyl-1,3-thiazol-3-ium-2-yl)diazen-1-yl]phenyl})amino]pentyl})amino]phenyl}diazen-1-yl)-1,3-thiazol-3-ium Sulfat
- 3-Methyl-2-(2-{4-[methyl({5-[methyl({4-[2-(3-methyl-1,3-thiazol-3-ium-2-yl)diazen-1-yl]phenyl})amino]pentyl})amino]phenyl}diazen-1-yl)-1,3-thiazol-3-ium Di(toluolsulfonat)
- 3-Methyl-2-(2-{4-[methyl({5-[methyl({4-[2-(3-methyl-1,3-thiazol-3-ium-2-yl)diazen-1-yl]phenyl})amino]pentyl})amino]phenyl}diazen-1-yl)-1,3-thiazol-3-ium Di(methylsulfat)
- 3-Methyl-2-(2-{4-[methyl({5-[methyl({4-[2-(3-methyl-1,3-thiazol-3-ium-2-yl)diazen-1-yl]phenyl})amino]pentyl})amino]phenyl}diazen-1-yl)-1,3-thiazol-3-ium Tetrachlorozinkat
- 2-(2-{4-[Ethyl({3-[ethyl({4-[2-(3-methyl-1,3-thiazo1-3-ium-2-yl)diazen-1-yl]phenyl})amino]propyl})amino]phenyl}diazen-1-yl)-3-methyl-1,3-thiazol-3-ium Dichlorid
- 2-(2-{4-[Ethyl({3-[ethyl({4-[2-(3-methyl-1,3-thiazol-3-ium-2-yl)diazen-1-yl]phenyl})amino]propyl})amino]phenyl}diazen-1-yl)-3-methyl-1,3-thiazol-3-ium Dibromid
- 2-(2-{4-[Ethyl({3-[ethyl({4-[2-(3-methyl-1,3-thiazol-3-ium-2-yl)diazen-1-yl]phenyl})amino]propyl})amino]phenyl}diazen-1-yl)-3-methyl-1,3-thiazol-3-ium Sulfat
- 2-(2-{4-[Ethyl({3-[ethyl({4-[2-(3-methyl-1,3-thiazol-3-ium-2-yl)diazen-1-yl]phenyl})amino]propyl})amino]phenyl}diazen-1-yl)-3-methyl-1,3-thiazol-3-ium Di(toluolsulfonat)
- 2-(2-{4-[Ethyl({3-[ethyl({4-[2-(3-methyl-1,3-thiazol-3-ium-2-yl)diazen-1-yl]phenyl})amino]propyl})amino]phenyl}diazen-1-yl)-3-methyl-1,3-thiazol-3-ium Di(methylsulfat)
- 2-(2-{4-[Ethyl({3-[ethyl({4-[2-(3-methyl-1,3-thiazol-3-ium-2-yl)diazen-1-yl]phenyl})amino]propyl})amino]phenyl}diazen-1-yl)-3-methyl-1,3-thiazol-3-ium Tetrachlorozinkat
- 2-(2-{4-[Ethyl({4-[ethyl({4-[2-(3-methyl-1,3-thiazol-3-ium-2-yl)diazen-1-yl]phenyl})amino]butyl})amino]phenyl}diazen-1-yl)-3-methyl-1,3-thiazol-3-ium Dichlorid
- 2-(2-{4-[Ethyl({4-[ethyl({4-[2-(3-methyl-1,3-thiazol-3-ium-2-yl)diazen-1-yl]phenyl})amino]butyl})amino]phenyl}diazen-1-yl)-3-methyl-1,3-thiazol-3-ium Dibromid
- 2-(2-{4-[Ethyl({4-[ethyl({4-[2-(3-methyl-1,3-thiazol-3-ium-2-yl)diazen-1-yl]phenyl})amino]butyl})amino]phenyl}diazen-1-yl)-3-methyl-1,3-thiazol-3-ium Sulfat
- 2-(2-{4-[Ethyl({4-[ethyl({4-[2-(3-methyl-1,3-thiazol-3-ium-2-yl)diazen-1-yl]phenyl})amino]butyl})amino]phenyl}diazen-1-yl)-3-methyl-1,3-thiazol-3-ium Di(toluolsulfonat)
- 2-(2-{4-[Ethyl({4-[ethyl({4-[2-(3-methyl-1,3-thiazol-3-ium-2-yl)diazen-1-yl]phenyl})amino]butyl})amino]phenyl}diazen-1-yl)-3-methyl-1,3-thiazol-3-ium Di(methylsulfat)
- 2-(2-{4-[Ethyl({4-[ethyl({4-[2-(3-methyl-1,3-thiazol-3-ium-2-yl)diazen-1-yl]phenyl})amino]butyl})amino]phenyl}diazen-1-yl)-3-methyl-1,3-thiazol-3-ium Tetrachlorozinkat
- 2-(2-{4-[Ethyl({5-[ethyl({4-[2-(3-methyl-1,3-thiazol-3-ium-2-yl)diazen-1-yl]phenyl})amino]pentyl})amino]phenyl}diazen-1-yl)-3-methyl-1,3-thiazol-3-ium Dichlorid
- 2-(2-{4-[Ethyl({5-[ethyl({4-[2-(3-methyl-1,3-thiazol-3-ium-2-yl)diazen-1-yl]phenyl})amino]pentyl})amino]phenyl}diazen-1-yl)-3-methyl-1,3-thiazol-3-ium Dibromid
- 2-(2-{4-[Ethyl({5-[ethyl({4-[2-(3-methyl-1,3-thiazol-3-ium-2-yl)diazen-1-yl]phenyl})amino]pentyl})amino]phenyl}diazen-1-yl)-3-methyl-1,3-thiazol-3-ium Sulfat
- 2-(2-{4-[Ethyl({5-[ethyl({4-[2-(3-methyl-1,3-thiazol-3-ium-2-yl)diazen-1-yl]phenyl})amino]pentyl})amino]phenyl}diazen-1-yl)-3-methyl-1,3-thiazol-3-ium Di(toluolsulfonat)
- 2-(2-{4-[Ethyl({5-[ethyl({4-[2-(3-methyl-1,3-thiazol-3-ium-2-yl)diazen-1-yl]phenyl})amino]pentyl})amino]phenyl}diazen-1-yl)-3-methyl-1,3-thiazol-3-ium Di(methylsulfat)
- 2-(2-{4-[Ethyl({5-[ethyl({4-[2-(3-methyl-1,3-thiazol-3-ium-2-yl)diazen-1-yl]phenyl})amino]pentyl})amino]phenyl}diazen-1-yl)-3-methyl-1,3-thiazol-3-ium Tetrachlorozinkat
- 3-Ethyl-2-[2-(4-{[3-({4-[2-(3-ethyl-1,3-thiazol-3-ium-2-yl)diazen-1-yl]phenyl}amino)propyl]amino}phenyl)diazen-1-yl]-1,3-thiazol-3-ium Dichlorid
- 3-Ethyl-2-[2-(4-{[3-({4-[2-(3-ethyl-1,3-thiazol-3-ium-2-yl)diazen-1-yl]phenyl}amino)propyl]amino}phenyl)diazen-1-yl]-1,3-thiazol-3-ium Dibromid
- 3-Ethyl-2-[2-(4-{[3-({4-[2-(3-ethyl-1,3-thiazol-3-ium-2-yl)diazen-I-yl]phenyl}amino)propyl]amino}phenyl)diazen-1-yl]-1,3-thiazol-3-ium Sulfat
- 3-Ethyl-2-[2-(4-{[3-({4-[2-(3-ethyl-1,3-thiazol-3-ium-2-yl)diazen-1-yl]phenyl}amino)propyl]amino}phenyl)diazen-1-yl]-1,3-thiazol-3-ium Di(toluolsulfonat)
- 3-Ethyl-2-[2-(4-{[3-({4-[2-(3-ethyl-1,3-thiazol-3-ium-2-yl)diazen-1-yl]phenyl}amino)propyl]amino}phenyl)diazen-1-yl]-1,3-thiazol-3-ium Di(methylsulfat)
- 3-Ethyl-2-[2-(4-{[3-({4-[2-(3-ethyl-1,3-thiazol-3-ium-2-yl)diazen-1-yl]phenyl}amino)propyl]amino}phenyl)diazen-1-yl]-1,3-thiazol-3-ium Tetrachlorozinkat
- 3-Ethyl-2-[2-(4-{[4-({4-[2-(3-ethyl-1,3-thiazol-3-ium-2-yl)diazen-1-yl]phenyl}amino)butyl]amino}phenyl)diazen-1-yl]-1,3-thiazol-3-ium Dichlorid
- 3-Ethyl-2-[2-(4-{[4-({4-[2-(3-ethyl-1,3-thiazol-3-ium-2-yl)diazen-1-yl]phenyl}amino)butyl]amino}phenyl)diazen-1-yl]-1,3-thiazol-3-ium Dibromid
- 3-Ethyl-2-[2-(4-{[4-({4-[2-(3-ethyl-1,3-thiazol-3-ium-2-yl)diazen-1-yl]phenyl}amino)butyl]amino}phenyl)diazen-1-yl]-1,3-thiazol-3-ium Sulfat
- 3-Ethyl-2-[2-(4-{[4-({4-[2-(3-ethyl-1,3-thiazol-3-ium-2-yl)diazen-1-yl]phenyl}amino)butyl]amino}phenyl)diazen-1-yl]-1,3-thiazol-3-ium Di(toluolsulfonat)
- 3-Ethyl-2-[2-(4-{[4-({4-[2-(3-ethyl-1,3-thiazol-3-ium-2-yl)diazen-1-yl]phenyl}amino)butyl]amino}phenyl)diazen-1-yl]-1,3-thiazol-3-ium Di(methylsulfat)
- 3-Ethyl-2-[2-(4-{[4-({4-[2-(3-ethyl-1,3-thiazol-3-ium-2-yl)diazen-1-yl]phenyl}amino)butyl]amino}phenyl)diazen-1-yl]-1,3-thiazol-3-ium Tetrachlorozinkat
- 3-Ethyl-2-[2-(4-{[5-({4-[2-(3-ethyl-1,3-thiazol-3-ium-2-yl)diazen-1-yl]phenyl}amino)pentyl]amino}phenyl)diazen-1-yl]-1,3-thiazol-3-ium Dichlorid
- 3-Ethyl-2-[2-(4-{[5-({4-[2-(3-ethyl-1,3-thiazol-3-ium-2-yl)diazen-1-yl]phenyl}amino)pentyl]amino}phenyl)diazen-1-yl]-1,3-thiazol-3-ium Dibromid
- 3-Ethyl-2-[2-(4-{[5-({4-[2-(3-ethyl-1,3-thiazol-3-ium-2-yl)diazen-1-yl]phenyl}amino)pentyl]amino}phenyl)diazen-1-yl]-1,3-thiazol-3-ium Sulfat
- 3-Ethyl-2-[2-(4-{[5-({4-[2-(3-ethyl-1,3-thiazol-3-ium-2-yl)diazen-1-yl]phenyl}amino)pentyl]amino}phenyl)diazen-1-yl]-1,3-thiazol-3-ium Di(toluolsulfonat)
- 3-Ethyl-2-[2-(4-{[5-({4-[2-(3-ethyl-1,3-thiazol-3-ium-2-yl)diazen-1-yl]phenyl}amino)pentyl]amino}phenyl)diazen-1-yl]-1,3-thiazol-3-ium Di(methylsulfat)
- 3-Ethyl-2-[2-(4-{[5-({4-[2-(3-ethyl-1,3-thiazol-3-ium-2-yl)diazen-1-yl]phenyl}amino)pentyl]amino}phenyl)diazen-1-yl]-1,3-thiazol-3-ium Tetrachlorozinkat
- 3-Ethyl-2-[2-(4-{[3-({4-[2-(3-ethyl-1,3-thiazol-3-ium-2-yl)diazen-1-yl]phenyl}(methyl)amino)propyl](methyl)amino}phenyl)diazen-1-yl]-1,3-thiazol-3-ium Dichlorid
- 3-Ethyl-2-[2-(4-{[3-({4-[2-(3-ethyl-1,3-thiazol-3-ium-2-yl)diazen-1-yl]phenyl}(methyl)amino)propyl](methyl)amino}phenyl)diazen-1-yl]-1,3-thiazol-3-ium Dibromid
- 3-Ethyl-2-[2-(4-{[3-({4-[2-(3-ethyl-1,3-thiazol-3-ium-2-yl)diazen-1-yl]phenyl}(methyl)amino)propyl](methyl)amino}phenyl)diazen-1-yl]-1,3-thiazol-3-ium Sulfat
- 3-Ethyl-2-[2-(4-{[3-({4-[2-(3-ethyl-1,3-thiazol-3-ium-2-yl)diazen-1-yl]phenyl}(methyl)amino)propyl]-(methyl)amino}phenyl)diazen-1-yl]-1,3-thiazol-3-ium Di(toluolsulfonat)
- 3-Ethyl-2-[2-(4-{[3-({4-[2-(3-ethyl-1,3-thiazol-3-ium-2-yl)diazen-1-yl]phenyl)-(methyl)amino)propyl](methyl)amino}phenyl)diazen-1-yl]-1,3-thiazol-3-ium Di(methylsulfat)
- 3-Ethyl-2-[2-(4-{[3-({4-[2-(3-ethyl-1,3-thiazol-3-ium-2-yl)diazen-1-yl]phenyl}-(methyl)amino)propyl](methyl)amino}phenyl)diazen-1-yl]-1,3-thiazol-3-ium Tetrachlorozinkat
- 3-Ethyl-2-[2-(4-{[4-({4-[2-(3-ethyl-1,3-thiazol-3-ium-2-yl)diazen-1-yl]phenyl}(methyl)amino)butyl](methyl)amino}phenyl)diazen-1-yl]-1,3-thiazol-3-ium Dichlorid
- 3-Ethyl-2-(2-(4-{[4-({4-[2-(3-ethyl-1,3-thiazol-3-ium-2-yl)diazen-1-yl]phenyl}(methyl)amino)butyl](methyl)amino}phenyl)diazen-1-yl]-1,3-thiazol-3-ium Dibromid
- 3-Ethyl-2-[2-(4-{[4-({4-[2-(3-ethyl-1,3-thiazol-3-ium-2-yl)diazen-1-yl]phenyl}(methyl)amino)butyl](methyl)aminolphenyl)diazen-1-yl]-1,3-thiazol-3-ium Sulfat
- 3-Ethyl-2-[2-(4-{[4-({4-[2-(3-ethyl-1,3-thiazol-3-ium-2-yl)diazen-1-yl]phenyl)-(methyl)amino)butyl](methyl)amino}phenyl)diazen-1-yl]-1,3-thiazol-3-ium Di(toluolsulfonat)
- 3-Ethyl-2-[2-(4-{[4-({4-[2-(3-ethyl-1,3-thiazol-3-ium-2-yl)diazen-1-yl]phenyl)-(methyl)amino)butyl](methyl)amino}phenyl)diazen-1-yl]-1,3-thiazol-3-ium Di(methylsulfat)
- 3-Ethyl-2-[2-(4-{[4-({4-[2-(3-ethyl-1,3-thiazol-3-ium-2-yl)diazen-1-yl]phenyl}-(methyl)amino)butyl](methyl)amino}phenyl)diazen-1-yl]-1,3-thiazol-3-ium
- 3-Ethyl-2-[2-(4-{[5-({4-[2-(3-ethyl-1,3-thiazol-3-ium-2-yl)diazen-1-yl]phenyl}(methyl)amino)pentyl](methyl)amino}phenyl)diazen-1-yl]-1,3-thiazol-3-ium Dichlorid
- 3-Ethyl-2-[2-(4-{[5-({4-[2-(3-ethyl-1,3-thiazol-3-ium-2-yl)diazen-1-yl]phenyl}(methyl)amino)pentyl](methyl)amino}phenyl)diazen-1-yl]-1,3-thiazol-3-ium Dibromid
- 3-Ethyl-2-[2-(4-{[5-({4-[2-(3-ethyl-1,3-thiazol-3-ium-2-yl)diazen-1-yl]phenyl}(methyl)amino)pentyl](methyl)amino}phenyl)diazen-1-yl]-1,3-thiazol-3-ium Sulfat
- 3-Ethyl-2-[2-(4-{[5-({4-[2-(3-ethyl-1,3-thiazol-3-ium-2-yl)diazen-1-yl]phenyl}-(methyl)amino)pentyl](methyl)amino}phenyl)diazen-1-yl]-1,3-thiazol-3-ium Di(toluolsulfonat)
- 3-Ethyl-2-[2-(4-{[5-({4-[2-(3-ethyl-1,3-thiazol-3-ium-2-yl)diazen-1-yl]phenyl}-(methyl)amino)pentyl](methyl)amino}phenyl)diazen-1-yl]-1,3-thiazol-3-ium Di(methylsulfat)
- 3-Ethyl-2-[2-(4-{[5-({4-[2-(3-ethyl-1,3-thiazol-3-ium-2-yl)diazen-1-yl]phenyl}-(methyl)amino)pentyl](methyl)amino}phenyl)diazen-1-yl]-1,3-thiazol-3-ium Tetrachlorozinkat
- 3-Ethyl-2-(2-{4-[ethyl({3-[ethyl({4-[2-(3-ethyl-1,3-thiazol-3-ium-2-yl)diazen-1-yl]phenyl})amino]propyl})amino]phenyl}diazen-1-yl)-1,3-thiazol-3-ium Dichlorid
- 3-Ethyl-2-(2-{4-[ethyl({3-[ethyl({4-[2-(3-ethyl-1,3-thiazol-3-ium-2-yl)diazen-1-yl]phenyl})amino]propyl})amino]phenyl}diazen-1-yl)-1,3-thiazol-3-ium Dibromid
- 3-Ethyl-2-(2-{4-[ethyl({3-[ethyl({4-[2-(3-ethyl-1,3-thiazol-3-ium-2-yl)diazen-1-yl]phenyl})amino]propyl})amino]phenyl}diazen-1-yl)-1,3-thiazol-3-ium Sulfat
- 3-Ethyl-2-(2-{4-[ethyl({3-[ethyl({4-[2-(3-ethyl-1,3-thiazol-3-ium-2-yl)diazen-1-yl]phenyl})amino]propyl})amino]phenyl}diazen-1-yl)-1,3-thiazol-3-ium Di(toluolsulfonat)
- 3-Ethyl-2-(2-{4-[ethyl({3-[ethyl({4-[2-(3-ethyl-1,3-thiazo1-3-ium-2-yl)diazen-1-yl]phenyl})amino]propyl})amino]phenyl}diazen-1-yl)-1,3-thiazol-3-ium Di(methylsulfat)
- 3-Ethyl-2-(2-{4-[ethyl({3-[ethyl({4-[2-(3-ethyl-1,3-thiazol-3-ium-2-yl)diazen-1-yl]phenyl})amino]propyl})amino]phenyl}diazen-1-yl)-1,3-thiazol-3-ium Tetrachlorozinkat
- 3-Ethyl-2-(2-{4-[ethyl({4-[ethyl({4-[2-(3-ethyl-1,3-thiazol-3-ium-2-yl)diazen-1-yl]phenyl})amino]butyl})amino]phenyl}diazen-1-yl)-1,3-thiazol-3-ium Dichlorid
- 3-Ethyl-2-(2-{4-[ethyl({4-[ethyl({4-[2-(3-ethyl-1,3-thiazol-3-ium-2-yl)diazen-1-yl]phenyl})amino]butyl})amino]phenyl}diazen-1-yl)-1,3-thiazol-3-ium Dibromid
- 3-Ethyl-2-(2-{4-[ethyl({4-[ethyl({4-[2-(3-ethyl-1,3-thiazol-3-ium-2-yl)diazen-1-yl]phenyl})amino]butyl})amino]phenyl}diazen-1-yl)-1,3-thiazol-3-ium Sulfat
- 3-Ethyl-2-(2-{4-[ethyl({4-[ethyl({4-[2-(3-ethyl-1,3-thiazol-3-ium-2-yl)diazen-1-yl]phenyl})amino]butyl})amino]phenyl}diazen-1-yl)-1,3-thiazol-3-ium Di(toluolsulfonat)
- 3-Ethyl-2-(2-{4-[ethyl({4-[ethyl({4-[2-(3-ethyl-1,3-thiazol-3-ium-2-yl)diazen-1-yl]phenyl})amino]butyl})amino]phenyl}diazen-1-yl)-1,3-thiazol-3-ium Di(methylsulfat)
- 3-Ethyl-2-(2-{4-[ethyl({4-[ethyl({4-[2-(3-ethyl-1,3-thiazol-3-ium-2-yl)diazen-1-yl]phenyl})amino]butyl})amino]phenyl}diazen-1-yl)-1,3-thiazol-3-ium Tetrachlorozinkat
- 3-Ethyl-2-(2-{4-[ethyl({5-[ethyl({4-[2-(3-ethyl-1,3-thiazol-3-ium-2-yl)diazen-1-yl]phenyl})amino]pentyl})amino]phenyl}diazen-1-yl)-1,3-thiazol-3-ium Dichlorid
- 3-Ethyl-2-(2-{4-[ethyl({5-[ethyl({4-[2-(3-ethyl-1,3-thiazol-3-ium-2-yl)diazen-1-yl]phenyl})amino]pentyl})amino]phenyl}diazen-1-yl)-1,3-thiazol-3-ium Dibromid
- 3-Ethyl-2-(2-{4-[ethyl({5-[ethyl({4-[2-(3-ethyl-1,3-thiazol-3-ium-2-yl)diazen-1-yl]phenyl})amino]pentyl})amino]phenyl}diazen-1-yl)-1,3-thiazol-3-ium Sulfat
- 3-Ethyl-2-(2-{4-[ethyl({5-[ethyl({4-[2-(3-ethyl-1,3-thiazol-3-ium-2-yl)diazen-1-yl]phenyl})amino]pentyl})amino]phenyl}diazen-1-yl)-1,3-thiazol-3-ium Di(toluolsulfonat)
- 3-Ethyl-2-(2-{4-[ethyl({5-[ethyl({4-[2-(3-ethyl-1,3-thiazol-3-ium-2-yl)diazen-1-yl]phenyl})amino]pentyl})amino]phenyl}diazen-1-yl)-1,3-thiazol-3-ium Di(methylsulfat)
- 3-Ethyl-2-(2-{4-[ethyl({5-[ethyl({4-[2-(3-ethyl-1,3-thiazol-3-ium-2-yl)diazen-1-yl]phenyl})amino]pentyl})amino]phenyl}diazen-1-yl)-1,3-thiazol-3-ium Tetrachlorozinkat

Im Rahmen der zu dieser Erfindung führenden Arbeiten hat sich herausgestellt, dass mit den direktziehenden Farbstoffen (a) der Formel (I) besonders dann intensive Färbungen im Blaubereich erzielt werden konnten, wenn
- R1, R4: unabhängig voneinander für eine Methylgruppe oder eine Ethylgruppe stehen,
- R2, R3: unabhängig voneinander für eine Methylgruppe oder für eine Ethylgruppe stehen,
- R5, R6, R7, R8: jeweils für ein Wasserstoffatom stehen
- Q: für eine Gruppierung der Formel (II) steht,

*-(CH₂)n-* (II)

und
n für die Zahl 3 oder 4 steht.

Explizit ganz besonders bevorzugt sind daher erfindungsgemäße Mittel zum Färben von keratinischen Fasern, welche mindestens einen Farbstoff (a) der Formel (I) enthalten, der ausgewählt ist aus der Gruppe aus
- Salzen des 3-Methyl-2-(2-{4-[methyl({3-[methyl({4-[2-(3-methyl-1,3-thiazol-3-ium-2-yl)diazen-1-yl]phenyl})amino]propyl})amino]phenyl}diazen-1-yl)-1,3-thiazol-3-iums 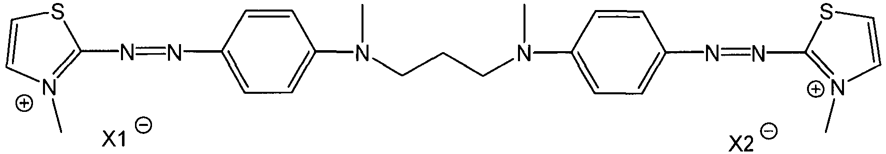
- Salzen des 3-Methyl-2-(2-{4-[methyl({4-[methyl({4-[2-(3-methyl-1,3-thiazol-3-ium-2-yl)diazen-1-yl]phenyl})amino]butyl})amino]phenyl}diazen-1-yl)-1,3-thiazol-3-iums 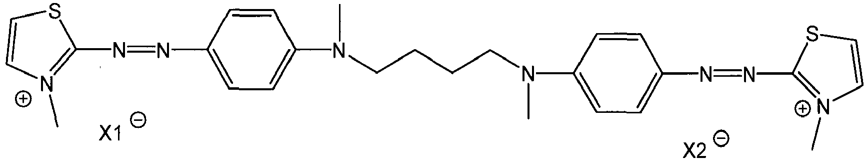
- Salzen des 2-(2-{4-[Ethyl({3-[ethyl({4-[2-(3-methyl-1,3-thiazol-3-ium-2-yl)diazen-1-yl]phenyl})amino]propyl})amino]phenyl}diazen-1-yl)-3-methyl-1,3-thiazol-3-iums 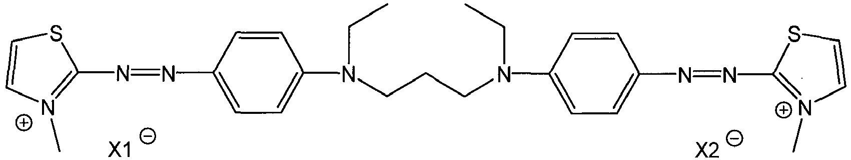
- Salzen des 2-(2-{4-[Ethyl({4-[ethyl({4-[2-(3-methyl-1,3-thiazol-3-ium-2-yl)diazen-1-yl]phenyl})amino]butyl})amino]phenyl}diazen-1-yl)-3-methyl-1,3-thiazol-3-iums 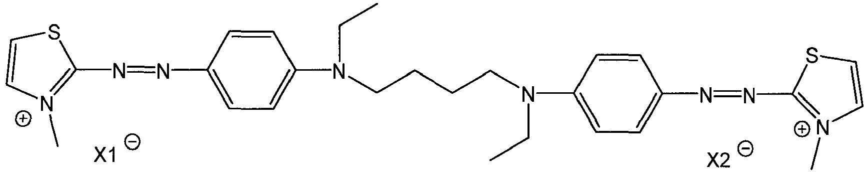
- Salzen des 3-Ethyl-2-[2-(4-{[3-({4-[2-(3-ethyl-1,3-thiazol-3-ium-2-yl)diazen-1-yl]phenyl}(methyl)amino)propyl](methyl)amino}phenyl)diazen-1-yl]-1,3-thiazol-3-iums 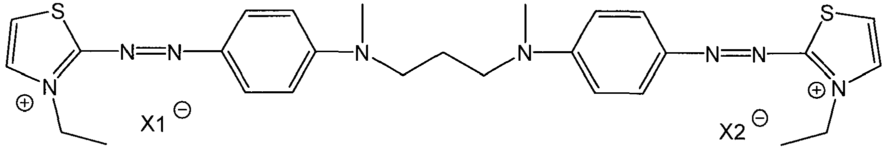
- Salzen des 3-ethyl-2-[2-(4-{[4-({4-[2-(3-ethyl-1,3-thiazol-3-ium-2-yl)diazen-1-yl]phenyl}(methyl)amino)butyl](methyl)amino}phenyl)diazen-1-yl]-1,3-thiazol-3-iums 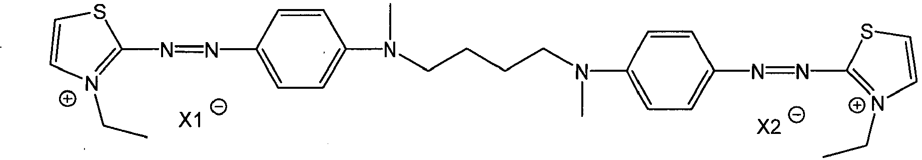
- Salzen des 3-Ethyl-2-(2-{4-[ethyl({3-[ethyl({4-[2-(3-ethyl-1,3-thiazol-3-ium-2-yl)diazen-1-yl]phenyl})amino]propyl})amino]phenyl}diazen-1-yl)-1,3-thiazol-3-iums 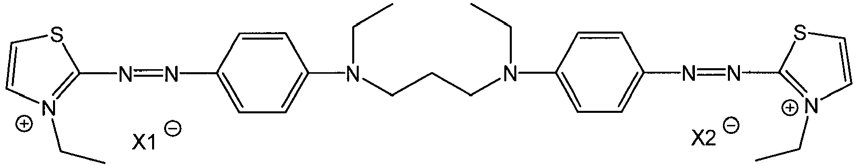
- Salzen des 3-Ethyl-2-(2-{4-[ethyl({4-[ethyl({4-[2-(3-ethyl-1,3-thiazol-3-ium-2-yl)diazen-1-yl]phenyl})amino]butyl})amino]phenyl}diazen-1-yl)-1,3-thiazol-3-iums

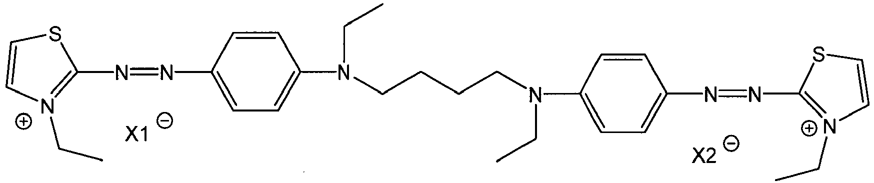

Die erfindungsgemäßen Mittel zum Färben von keratinischen Fasern enthalten den oder die direktziehenden Farbstoffe der Formel (I) vorzugsweise in einer Gesamtmenge von 0,01 bis 4,5 Gew.-%, bevorzugt von 0,05 bis 2,8 Gew.-%, weiter bevorzugt von 0,1 bis 2,2 Gew.-% und besonders bevorzugt von 0,2 bis 1,2 Gew.-% enthält. Die Mengenangabe in Gewichtsprozent bezieht sich hierbei auf die Gesamtmenge aller im Mittel enthaltenen Verbindungen der Formel (I), die zum Gesamtgewicht des Mittels in Relation gesetzt wird.

In einer weiteren bevorzugten Ausführungsform ist ein erfindungsgemäßes Mittel zum Färben von keratinischen Fasern daher dadurch gekennzeichnet, dass es - bezogen auf das Gesamtgewicht des Mittels- einen oder mehrere direktziehende Farbstoffe (a) der Formel (I) in einer Gesamtmenge von 0,01 bis 4,5 Gew.-%, bevorzugt von 0,05 bis 2,8 Gew.-%, weiter bevorzugt von 0,1 bis 2,2 Gew.-% und besonders bevorzugt von 0,2 bis 1,2 Gew.-% enthält.

Die Farbstoffe der allgemeinen Formel (I) können beispielsweise nach einem Verfahren hergestellt werden, wie es in WO 2002/100369 A2 beschrieben ist.

So kann beispielsweise das Edukt 2-Aminothiazol in konzentrierter Schwefelsäure mit Nitrosylschwefelsäure in das Diazoniumion überführt werden:

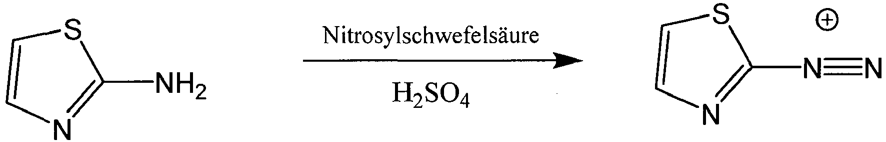

Das reaktive Diazoniumion geht dann mit dimeren Anilin-Derivaten eine doppelte Azokupplungsreaktion ein:

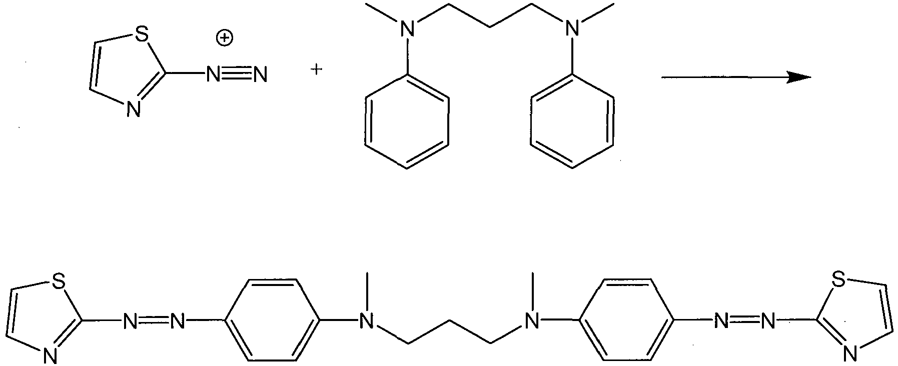

Der in der Azokupplungsreaktion entstehende neutrale dimere Farbstoff kann dann schließlich mit Quaternierungsmitteln doppelt quaterniert werden. Die Quaternierungsreaktion wird bevorzugt in einem polaren aprotischen Lösungsmittel (wie beispielsweise DMSO, DMF etc.) durchgeführt. Als Quaternierungsmittel kommen zum Beispiel Dimethylsulfat, Methyliodid oder p-Toluolsulfonat in Frage.

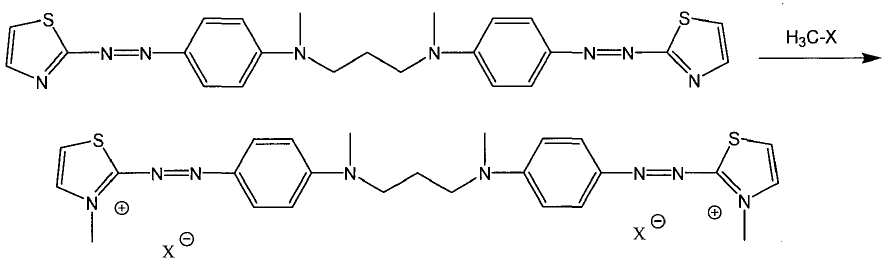

Als zweite erfindungswesentliche Komponente (b) enthalten die Mittel zum Färben von keratinischen Fasern mindestens ein nichtionisches Tensid.

Tenside sind amphiphile (bifunktionelle) Verbindungen, die aus mindestens einem hydrophoben und mindestens einem hydrophilen Molekülteil bestehen. Bei dem hydrophoben Rest handelt es sich bevorzugt eine Kohlenwasserstoffkette mit 12-30 Kohlenstoff-Atomen, die gesättigt oder ungesättigt, linear oder verzweigt sein kann. Besonders bevorzugt ist diese C₁₂-C₃₀-Alkylkette linear. Bei nichtionischen Tensiden umfasst der hydrophile Molekülteil eine ungeladene hydrophile Kopfgruppe, welche starke Dipolmomente aufweist und in wässriger Lösung stark hydratisiert ist.

Nichtionische Tenside enthalten als hydrophile Gruppe z.B. eine Polyolgruppe, eine Polyalkylenglykolether oder eine Kombination aus Polyol und Polyglycolethergruppe. Solche Verbindungen sind beispielsweise
- Anlagerungsprodukte von 2 bis 100 Mol Ethylenoxid und/oder 2 bis 100 Mol Propylenoxid an linaere und verzweigte Fettalkohole mit 12 bis 30 C-Atomen, die Fettalkoholpolyglykolether bzw. die Fettalkoholpolypropylenglycolether bzw. gemischte Fettalkoholpolyether,
- Anlagerungsprodukte von 2 bis 100 Mol Ethylenoxid und/oder 2 bis 100 Mol Propylenoxid an lineare und verzweigte Fettsäuren mit 12 bis 30 C-Ateomen, die Fettsäurepolyglykolether bzw. die Fettsäurepolypropylenglykolether bzw. gewmischte Fettsäurepolyether,
- Anlagerungsprodukte von 2 bis 50 Mol Ethylenoxid und/oder 2 bis 50 Mol Propylenoxid an lineare und verzweigte Alkylphenole mit 8 bis 15 C-Atomen in der Alkylgruppe, die Alkylphenolpolyglykolether bzw. die Alkylphenolpolypropylenglykolether bzw. gemischte Alkylphenolpolyether,
- mit einem Methyl- oder C2-C6-Alkylrest endgruppen verschlossene Anlagerungsprodukt von 2 bis 50 Mol Ethylenoxid und/oder 1 bis 5 mol Propylenoxid an lineare und verzweigte Fettalkohole mit 12 bis 30 C-Atomen, an Fettsäuren mit 8 bis 30 C-Atomen und an Alkylphenole mit 8 bis 15 C-Atomen in der Alkylgruppe,
- C12-C30-Fettsäuremono- und -diester von Alagerungsrpodukten von 1 bis 30 Mol Ethylenoxid an Glycerin
- Anlagerungsprodukte von 5 bis 60 Mol Ethylenoxid an Rizinusöl und hydriertes Rizinusöl,
- alkoxylierte Triglyceride,
- alkoxylierte Fettsäurealkylester der Formel (Tnio-1)

   R¹CO-(OCH₂CHR²)_{w}OR³ (Tnio-1)

   in der
   R1-CO für einen linearen oder verzweigten, gesättigten und/oder ungesättigten Acylrest mit 12 bis 30 C-Atomen,
   R2 für Wasserstoff oder Methyl,
   R3 für einen linearen oder verzweigten C₁-C₄-Alkylrest und
   w für die Zahlen 1 bis 20 steht,
- Aminoxide,
- Sorbitanfettsäureester und Anlagerungsprodukte von Ethylenoxid an Sorbitanfettsäureester wie beispielsweise Polysorbate,
- Zuckerfettsäureester und Anlagerungsprodukte von Ethylenoxid an Zuckerfettsäureester,
- Anlagerungsprodukte von Ethylenoxid an Fettsäurealkanolamide un
- Zuckertenside der Formel (Tnio-2)

   R⁴O-[G]ₚ (Tnio-2)

   in der
   R2 für einen linearen C₁₂-C₃₀-Alkylest steht,
   G für einen Zuckerrest mit 5 oder 6 Kohlenstoffatomen, bevorzugt für Glucose, steht und
   p für eine ganze Zahl von 1 bis 10, bevorzugt für eine ganze Zahl von 1 bis 6, steht.

Fettalkohole sind explizit keine nichtionischen Tenside im Sinne der vorliegenden Erfindung. Fettalkohole im Sinne der vorliegenden Erfindung fallen in die Gruppe der Fettstoffe.

Die Behandlung von keratinischen Fasern mit Mitteln, die (a) mindestens einen direktziehenden Farbstoff der Formel (I) und (b) mindestens ein nichtionisches Tensid enthielten, führte zu intensiven Färbungen in attraktiven, reinen Blaunuancen ohne Rotanteil. Hierbei hat sich überraschenderweise gezeigt, dass das Farbaufzugsvermögen durch Einsatz eines oder mehrere spezieller nichtionischer Tenside noch weiter optimiert werden konnte. Besonders intensive Blaufärbungen wurden erhalten, wenn die Farbstoffe (a) der Formel (I) mit mindestens einem nichtionischen Tensid (b) aus der Gruppe der
- Anlagerungsprodukte von 2 bis 100 Mol Ethylenoxid und/oder 2 bis 100 Mol Propylenoxid an lineare und verzweigte Fettalkohole mit 12 bis 30 C-Atomen, die Fettalkoholpolyglykolether bzw. die Fettalkoholpolypropylenglycolether bzw. gemischte Fettalkoholpolyether,
- Anlagerungsprodukte von 2 bis 100 Mol Ethylenoxid und/oder 2 bis 100 Mol Propylenoxid an lineare und verzweigte Fettsäuren mit 12 bis 30 C-Atomen, die Fettsäurepolyglykolether bzw. die Fettsäurepolypropylenglykolether bzw. gewmischte Fettsäurepolyether, und der
- Anlagerungsprodukte von 5 bis 60 Mol Ethylenoxid an Rizinusöl und hydriertes Rizinusöl.

In einer besonders bevorzugten Ausführungsform ist ein erfindungsgemäßes Mittel daher dadurch gekennzeichnet, dass es (b) mindestens ein nichtionisches Tensid enthält, das ausgewählt ist aus den
- Anlagerungsprodukten von 2 bis 100 Mol Ethylenoxid und/oder 2 bis 100 Mol Propylenoxid an lineare und verzweigte Fettalkohole mit 12 bis 30 C-Atomen, die Fettalkoholpolyglykolether bzw. die Fettalkoholpolypropylenglycolether bzw. gemischte Fettalkoholpolyether,
- Anlagerungsprodukte von 2 bis 100 Mol Ethylenoxid und/oder 2 bis 100 Mol Propylenoxid an lineare und verzweigte Fettsäuren mit 12 bis 30 C-Atomen, die Fettsäurepolyglykolether bzw. die Fettsäurepolypropylenglykolether bzw. gemischte Fettsäurepolyether, und der
- Anlagerungsprodukte von 5 bis 60 Mol Ethylenoxid an Rizinusöl und hydriertes Rizinusöl.

Besonders bevorzugte Anlagerungsrpodukte von 5 bis 60 Mol Ethylenoxid an Rizinusöl und hydriertes Rizinusöl sind beispielsweise unter den INCI Namen
- PEG-30 Castor Oil mit der CAS-Nummer 61791-12-6
- PEG-40 Hydrogenated Castor Oil mit der CAS-Nummer 61788-85-0
bekannt.

Unter den Anlagerungsprodukten von 2 bis 100 Mol Ethylenoxid an linaere und verzweigte Fettalkohole mit 12 bis 30 C-Atomen wird beispielsweise verstanden, dass jedes Mol Fettalkohol mit 2 bis 100 Mol Ethylenoxid umgesetzt wurde.

Bei den Anlagerungsprodukten von 2 bis 100 Mol Ethylenoxid an C12-C30-Fettalkohole handelt es sich demnach um Verbindungen der allgemeinen Formel (B1) 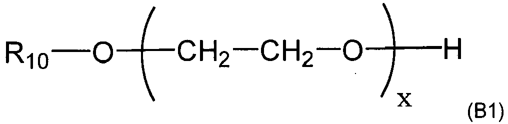 wobei
- R10: für eine lineare oder verzweigte C₁₂-C₃₀-Alkylgruppe steht und
- x: für eine ganze Zahl von 2 bis 100 steht.

Bei den Anlagerungsprodukten von 2 bis 100 Mol Ethylenoxid an lineare und verzweigte Fettsäuren mit 12 bis 30 C-Atomen, handelt es sich um Verbindungen der allgemeinen Formel (B2) 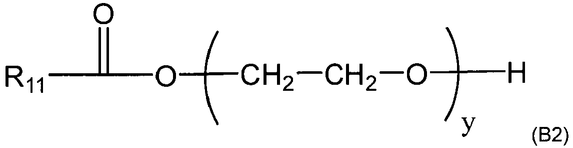 wobei
- R11: für eine lineare oder verzweigte C₁₁-C₂₉-Alkylgruppe steht
- y: für eine ganze Zahl von 2 bis 100 steht.

Innerhalb der Gruppe der getesteten nichtionischen Tenside konnten mit den Verbindungen der allgemeinen Formeln (B1) und/oder (B2) die stärkste Erhöhung der Farbintensität beobachtet werden, wenn diese nichtionischen Tenside in Kombination mit mindestens einem Farbstoff der allgemeinen Formel (I) ausgefärbt wurden.

In einer besonders bevorzugten Ausführungsform ist ein erfindungsgemäßes Mittel daher dadurch gekennzeichnet, dass es als nichtionisches Tensid (b) mindestens eine Verbindung der Formel (B1) und/oder mindestens eine Verbindung der Formel (B2) enthält, 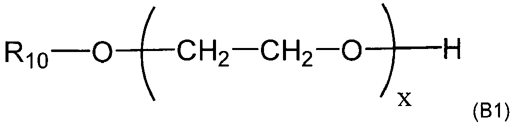 wobei
- R10: für eine lineare oder verzweigte C₁₂-C₃₀-Alkylgruppe steht und
- x: für eine ganze Zahl von 2 bis 100 steht, 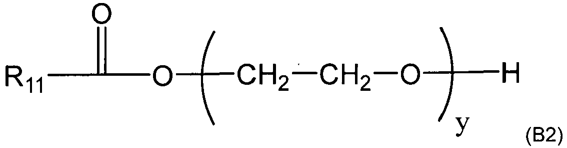
wobei
- R11: für eine lineare oder verzweigte C₁₁-C₂₉-Alkylgruppe steht und
- y: für eine ganze Zahl von 2 bis 100 steht.

Das allerbeste Aufzugsvermögen konnte beobachtet werden, wenn als nichtionisches Tensid (b) eine Verbindung der Formel (B1) eingesetzt wurde, in der x für eine ganze Zahl von 10 bis 50 steht.

In einer explizit ganz besonders bevorzugten Ausführungsform ist ein erfindungsgemäßes Mittel daher dadurch gekennzeichnet, dass es als nichtionisches Tensid (b) mindestens eine Verbindung der Formel (B1) enthält, 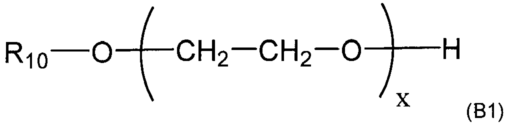 wobei
- R10: für eine lineare oder verzweigte C₁₂-C₃₀-Alkylgruppe steht und
- x: für eine ganze Zahl von 10 bis 50 steht.

Besonders bevorzugte Tenside dieser Ausführungsform sind beispielsweise unter den INCI Namen
- Ceteareth-12 (C16/C18 Fettalkohole, ethoxyliert mit 12 EO)
- Ceteareth-20 (C16/C18 Fettakohole, ethoxyliert mit 20 EO)
- Ceteareth-30 (C16/C18 Fettalkohole, ethoxyliert mit 30 EO)
bekannt.

Bevorzugt enthalten die erfindungsgemäßen Mittel zum Färben von keratinischen Fasern ein oder mehrere nichtionische Tenside (b) in einer Gesamtmenge von 0,2 bis 10,5 Gew.-%, bevorzugt von 1,0 bis 7,5 Gew.-%, weiter bevorzugt von 2,0 bis 4,5 Gew.-% und ganz besonders bevorzugt von 2,5 bis 4,5 Gew.-%. Hierbei sind die Angaben in Gew.-% auf die Gesamtmenge aller nichtionischen Tenside (b) bezogen, die zur Gesamtmenge des Färbemittels in Relation gesetzt werden.

In einer weiteren besonders bevorzugten Ausführungsform ist ein erfindungsgemäßes Mittel zum Färben von keratinischen Fasern daher dadurch gekennzeichnet, dass es - bezogen auf das Gesamtgewicht des Mittels - ein oder mehrere nichtionische Tenside (b) in einer Gesamtmenge von 0,2 bis 10,5 Gew.-%, bevorzugt von 1,0 bis 7,5 Gew.-%, weiter bevorzugt von 2,0 bis 4,5 Gew.-% und ganz besonders bevorzugt von 2,5 bis 4,5 Gew.-% enthält.

Weiterhin können die erfindungsgemäßen Mittel auch ein oder mehrere kationische Tenside enthalten. Unter kationischen Tensiden werden Tenside, also grenzflächenaktive Verbindungen, mit jeweils einer oder mehreren positiven Ladungen verstanden. Kationische Tenside enthalten ausschließlich positive Ladungen. Üblicherweise sind diese Tenside aus einem hydrophoben Teil und einer hydrophilen Kopfgruppe aufgebaut, wobei der hydrophobe Teil in der Regel aus einem Kohlenwasserstoff-Gerüst (z.B. bestehend aus einer oder zwei linearen oder verzweigten Alkylketten) besteht, und die positive(n) Ladung(en) in der hydrophilen Kopfgruppe lokalisiert sind. Kationische Tenside adsorbieren and Grenzflächen und aggregieren in wässriger Lösung oberhalb der kritischen Micellbildungskonzentration zu positiv geladenen Micellen.

Beispiele für Kationtenside sind
- quartäre Ammniumverbindungen, die als hydrophobe Reste ein oder zwei Alkylketten mit einer Kettenlänge von 8 bis 28 C-Atomen tragen können
- quartäre Phosphoniumsalze, substituiert mit einer oder mehreren Alkylketten mit einer einer Kettenlänge von 8 bis 28 C-Atomen oder
- tertiäre Sulfonium-Salze.

Weiterhin kann die kationische Ladung auch in Form einer Onium-Struktur Bestandteil eines heterozyklischen Ringes (z.B. eines Imidazoliumringe oder einer Pyridiniumringes) sein.

Neben der funktionellen Einheit, welche die kationische Ladung trägt, kann das Kationtensid auch weitere ungeladene funktionelle Gruppen beinhalten, diese ist beispielsweise bei Esterquats der Fall. Geeignete kationische Tenside dieses Typs sind beispielsweise physiologisch verträgliche Salze des N,N,N-Trimethyl-1-hexadecanaminiums, insbesondere N,N,N-Trimethyl-1-hexadecanaminiumchlorid, welches auch unter dem Handelsnamen Dehyquart A-CA vertrieben wird.

Bei einem weiteren geeigneten kationischen Tensid handelt es sich um ein physiologisch verträgliches Salze des Dimethyl-distearyldimethylammoniums, besonders bevorzugt um Dimethyldistearylammoniumchlorid.

Weitere kationische Tenside können aus der Gruppe der kationischen Imidazoliumverbindungen ausgewählt werden.

Das erfindungsgemäße Mittel kann das bzw. die kationischen Tenside in einer Gesamtmenge von 0,1 bis 4,8 Gew.-%, bevorzugt von 0,2 bis 2,4 Gew.-%, weiter bevorzugt von 0,3 bis 1,8 Gew.-%- bezogen auf das Gesamtgewicht des Mittels - enthalten.

Erfindungsgemäß geeignete Mittel können weiterhin dadurch gekennzeichnet sein, dass sie zusätzlich mindestens ein zwitterionisches Tensid enthalten. Geeignete zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Erfindungsgemäß geeignete Mittel sind dadurch gekennzeichnet, dass das Mittel zusätzlich mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und C₁₂-C₁₈-Acylsarcosin.

In einer weiteren bevorzugten Ausführungsform enthalten die erfindungsgemäßen Mittel neben der Verbindung der Formel (I) zusätzlich mindestens einen weiteren direktziehenden Farbstoff. Durch die Kombination mit weiteren kationischen direktziehenden Farbstoffen lässt sich das erzielbare Nuancenspektrum erweitern, und die Färbeeigenschaften lassen sich noch weiter verbessern. Direktziehende Farbstoffe können in anionische, kationische und nichtionische direktziehende Farbstoffe unterteilt werden.

Die direktziehenden Farbstoffe sind vorzugsweise ausgewählt aus den kationischen direktziehenden Farbstoffen, da diese mit den Farbstoffen der allgemeinen Formel (I) gut kompatibel sind.

In einer weiteren besonders bevorzugten Ausführungsform ist ein erfindungsgemäßes Mittel dadurch gekennzeichnet, dass es zusätzlich mindestens einen weiteren kationischen direktziehenden Farbstoff enthält, der von den Farbstoffen der Formel (I) verschieden ist.

Unter kationischen Farbstoffen werden in diesem Zusammenhang Farbstoffe verstanden, die mindestens eine positive Ladung tragen.

Als besonders gut kompatibel haben sich ein oder mehrere Farbstoffe aus der Gruppe Basic Yellow 87, Basic Orange 31, Basic Red 51, Basic Violet 2 und Cationic Blue 347 erwiesen.

Ganz besonders gut kompatibel sind die Farbstoffe der Formel (I) mit den kationischen Azofarbstoffen Basic Orange 31 und Basic Red 51. Durch Kombination eines Farbstoffes der Formel (I) mit Basic Orange 31 und/oder Basice Red 51 können - außer rein gelben Nuancen - Nuancen im gesamten Farbspektrum erzeugt werden.

In einer weiteren besonders bevorzugten Ausführungsform ist ein erfindungsgemäßes Mittel dadurch gekennzeichnet, dass es zusätzlich Basic Orange 31 und/oder Basic Red 51 enthält.

Das erfindungsgemäße Mittel kann aber auch zusätzlich mindestens einen nichtionischen direktziehenden Farbstoff enthalten. Dieser kann ausgewählt sein aus der Gruppe HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethyl-amino-3-nitrobenzoesäure und 2-Chlor-6-ethylamino-4-nitrophenol.

Zusätzlich können auch anionische direktziehende Farbstoffe enthalten sein, die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten sind. Die erfindungsgemäßen Mittel können weiterhin auch zusammen mit Oxidationsfärbemitteln eingesetzt werden. Solche Oxidationsfärbemittel enthalten zusätzlich mindestens ein Oxidationsfarbstoffvorprodukt, bevorzugt zumindest ein Oxidationsfarbstoffvorprodukt vom Entwickler-Typ und mindestens ein Oxidationsfarbstoffvorprodukt vom Kuppler-Typ. Besonders geeignete Oxidationsfarbstoffvorprodukte vom Entwickler-Typ sind dabei ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)-phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind dabei ausgewählt aus der Gruppe, gebildet aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

Die zusätzlichen direktziehenden Farbstoffe, Entwicklerkomponenten und Kupplerkomponenten werden bevorzugt jeweils in einem Anteil von 0,0001 bis 5,0 Gew.-%, vorzugsweise 0,001 bis 3,5 Gew.-%, jeweils bezogen auf das anwendungsbereite Mittel, verwendet. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Soll die Färbung mit den erfindungsgemäßen direktziehenden Farbstoffen der Formel (I) und eine oxidative Aufhellung der Keratinfasern in einem Schritt erfolgen, so enthalten die erfindungsgemäßen Mittel zusätzlich ein Oxidationsmittel, bevorzugt Wasserstoffperoxid und/oder eines seiner festen Anlagerungsprodukte an organische oder anorganische Verbindungen.

In einer bevorzugten Ausführungsform wird Wasserstoffperoxid selbst als wässrige Lösung verwendet. Die Konzentration einer Wasserstoffperoxid-Lösung im erfindungsgemäßen Mittel wird einerseits von den gesetzlichen Vorgaben und andererseits von dem gewünschten Effekt bestimmt; vorzugsweise werden 6 bis 12 Gew.-%ige Lösungen in Wasser verwendet. Erfindungsgemäß bevorzugte anwendungsbereite Mittel des ersten Erfindungsgegenstands sind dadurch gekennzeichnet, dass sie, bezogen auf das Gesamtgewicht des anwendungsbereiten Mittels, 0,5 bis 20 Gew.-%, vorzugsweise 1 bis 12,5 Gew.-%, besonders bevorzugt 2,5 bis 10 Gew.-% und insbesondere 3 bis 6 Gew.-% Wasserstoffperoxid, jeweils bezogen auf das Gesamtgewicht des Mittels, enthalten.

In einer weiteren besonders bevorzugten Ausführungsform ist ein erfindungsgemäßes Mittel dadurch gekennzeichnet, dass es - bezogen auf das Gesamtgewicht des Mittels - 0,5 bis 12,5 Gew.-%, bevorzugt 2,5 bis 10 Gew.-% und insbesondere 3 bis 6 Gew.-% Wasserstoffperoxid enthält.

Zur Erzielung einer verstärkten Aufhell- und Bleichwirkung kann das Mittel weiterhin mindestens ein Peroxo-Salz enthalten. Geeignete Peroxo-Salze sind anorganische Peroxoverbindungen, bevorzugt ausgewählt aus der Gruppe, gebildet aus Ammoniumperoxodisulfat, Alkalimetallperoxodisulfaten, Ammoniumperoxomonosulfat, Alkalimetallperoxomonosulfaten, Alkalimetallperoxodiphosphaten und Erdalkalimetallperoxiden. Besonders bevorzuge sind Peroxodisulfate, insbesondere Ammoniumperoxodisulfat, Kaliumperoxodisulfat und Natriumperoxodisulfat.

In einer weiteren besonders bevorzugten Ausführungsform ist ein erfindungsgemäßes Mittel dadurch gekennezeichnet, dass es zusätzlich mindestens ein Persulfat aus der Gruppe Ammoniumperoxodisulfat, Kaliumperoxodisulfat und Natriumperoxodisulfat enthält.

Die Persulfate sind jeweils in einer Menge von 0,5 bis 20 Gew.-%, vorzugsweise 1 bis 12,5 Gew.-%, besonders bevorzugt 2,5 bis 10 Gew.-% und insbesondere 3 bis 6 Gew.-%, bezogen auf das Gesamtgewicht des anwendungsbereiten Mittels, in dem erfindungsgemäßen Mittel enthalten.

Das Färbe- und/oder Mattier-Mittel kann zur Verstärkung der Blondierwirkung weitere Bleichkraftverstärker enthalten, wie beispielsweise Tetraacetylethylendiamin (TAED), 1,5-Diacetyl-2,4-dioxo-hexahydro-1,3,5-triazin (DADHT), Tetraacetylglykoluril (TAGU), N-Nonanoylsuccinimid (NOSI), n-Nonanoyl- oder Isononanoyloxybenzolsulfonat (n- bzw. i-NOBS), Phthalsäureanhydrid, Triacetin, Ethylenglykoldiacetat und 2,5-Diacetoxy-2,5-dihydrofuran sowie Carbonatsalze bzw. Hydrogencarbonatsalze, insbesondere Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat, und stickstoffhaltige, heterocyclische Bleichkraftverstärker, wie 4-Acetyl-1-methylpyridinium-p-toluolsulfonat, 2-Acetyl-1-methylpyridinium-p-toluolsulfonat, sowie N-Methyl-3,4-dihydroisochinolinium-p-toluolsulfonat.

Zur weiteren Steigerung der Aufhellung kann der erfindungsgemäßen Zusammensetzung zusätzlich mindestens eine SiO₂-Verbindung, wie Kieselsäure oder Silicate, insbesondere Wassergläser, zugesetzt sein. Es kann erfindungsgemäß bevorzugt sein, die SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder.

Die Färbemittel können weiterhin zusätzliche Wirk-, Hilfs- und Zusatzstoffe enthalten, um die Färbeleistung zu verbessern und weitere gewünschte Eigenschaften der Mittel einzustellen.

Vorzugsweise werden die Färbemittel als flüssige Zubereitung bereitgestellt und den Mitteln daher gegebenenfalls zusätzlich eine weitere oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, zwitterionischen, amphoteren und nichtionischen Tensiden und Emulgatoren ausgewählt.

Die erfindungsgemäßen Färbemittel können weitere Hilfs- und Zusatzstoffe enthalten. So hat es sich als vorteilhaft erwiesen, wenn die Mittel mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind
- anionische, synthetische Polymere;
- kationische, synthetische Polymere;
- natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen;
- nichtionische, vollsynthetische Polymere, wie Polyvinylalkohol oder Polyvinylpyrrolidinon; sowie
- anorganische Verdickungsmittel, insbesondere Schichtsilikate wie beispielsweise Bentonit, besonders Smektite, wie Montmorillonit oder Hectorit.

Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des anwendungsbereiten Mittels zwischen 7 und 11, insbesondere zwischen 8 und 10,5, liegt. Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)-Alkalimetallmetasilikaten, (Erd-)Alkalimetallphosphaten und (Erd-)Alkalimetallhydrogenphosphaten, ausgewählt werden. Bevorzugte anorganische Alkalisierungsmittel sind Magnesiumcarbonat, Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein organisches Alkalisierungsmittel enthalten. Eine Ausführungsform des ersten Erfindungsgegenstands ist dadurch gekennzeichnet, dass das Mittel zusätzlich mindestens ein Alkalisierungsmittel enthält, welches ausgewählt ist aus der Gruppe, die gebildet wird aus Ammoniak, Alkanolaminen und basischen Aminosäuren, insbesondere aus Ammoniak, Monoethanolamin und Arginin oder seinen verträglichen Salzen.

Weiterhin hat es sich als vorteilhaft erweisen, wenn die Färbemittel, insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und-distearat sowie PEG-3-distearat; Pigmente sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der Anwendungsmischung, eingesetzt.

Mit den Färbemitteln, welche die erfindungsgemäßen direktziehenden Farbstoffe der allgemeinen Formel (I) enthalten, lassen sich keratinische Fasern in ausgesprochen attraktiven und intensiven Blautönen färben. Bei den Farbstoffen der allgemeinen Formel (I) handelt es sich um kationische dimere Azofarbstoffe. Hierbei hat sich überraschenderweise herausgestellt, dass die Farbintensität von kationischen Azofarbstoffen durch Zusatz eines oder mehrere nichtionischer Tenside (b) der Formel (B1) noch weiter gesteigert werden kann, 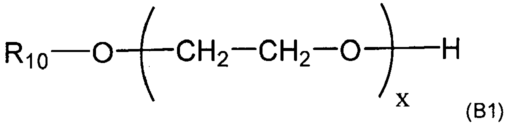 wobei
- R10: für eine lineare oder verzweigte C₁₂-C₃₀-Alkylgruppe steht und
- x: für eine ganze Zahl von 2 bis 100 steht.

Unter der Verbesserung des Farbaufzugsvermögens wird in diesem Zusammenhang verstanden, dass die Farbstoffe vermehrt bzw. verstärkt in die keratinische Faser hineindiffundieren, was zu Färbungen mit höherer Farbintensität führt. Die verstärkte Farbintensität lässt sich enweder visuell durch Betrachtung unter einer Tageslichtlampe oder aber durch farbmetrische Vermessung (Bestimmung der Lab-Werte) detektieren.

Geeigente kationische Azofarbstoffe sind beispielsweise die Farbstoffe Basic Yellow, Basic Orange 31 und Basic Red 51.

Die Intensivierung des Farbaufzugs durch die nichtionischen Tenside der Formel (B1) funktioniert zwar prinzipiell bei allen kationischen Azofarbstoffen. Besonders gut lässt sich durch Zugabe der nichtionischen Tenside der Formel (B1) jedoch der Farbaufzug der Farbstoffe der allgemeinen Formel (I) verbessern.

Die erfindungsgemäßen Mittel können als Einkomponentenmittel oder als Mehrkomponentenmittel wie Zweikomponenten Mittel oder Dreikomponentenmittel formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponente hergestellt.

Unter dem erfindungsgemäßen Mittel zur Farbveränderung von keratinischen Fasern wird immer das anwendungsbereite Mittel verstanden.

Wird das erfindungsgemäße Mittel dem Anwender in Form eines Einkomponenten-Mittels zur Verfügung gestellt, dann muss das anwendungsbereite Mittel nicht erst hergestellt werden, sondern kann direkt aus dem Behältnis, in dem es konfektioniert wurde, entnommen und auf die keratinischen Fasern appliziert werden.

Bei Blondiermitteln handelt es sich jedoch üblicherweise um Zweikomponentenprodukte, bei welchen eine oxidationsmittelhaltige Komponente (A1) kurz vor der Anwendung mit einem (Alkalisierungs-)Mittel (A2) vermischt und diese anwendungsbereite Mischung auf die Haare aufgetragen wird.

In diesem Fall handelt es sich bei dem erfindungsgemäßen Mittel um das anwendungsbereite Mittel, welches kurz vor der Anwendung durch Vermischen von (A1) und (A2) hergestellt wurde.

Hierbei können die direktziehenden Farbstoffe (a) der allgemeinen Formel (I) in der Komponente (A1) (d.h. zusammen mit dem Oxidationsmittel) oder aber in der Komponente (A2) (zusammen mit dem Alkalisierungsmittel) konfektioniert werden. Das bzw. die nichtionischen Tenside (b) können auch in der Komponente (A1) (d.h. zusammen mit dem Oxidationsmittel) oder aber in der Komponente (A2) (zusammen mit dem Alkalisierungsmittel) konfektioniert werden

Es ist ebenfalls möglich und erfindungsgemäß, wenn das anwendungsbereite Mittel kurz vor der Anwendung auf den menschlichen Haaren durch Vermischen von 3 Komponenten hergestellt wird, wobei
- die Komponente (A1) mindestens einen direktziehenden Farbstoff der allgemeinen Formel (I) und mindestens ein Alkalisierungsmittel
- die Komponente (A2) mindestens ein erstes Oxidationsmittel (z.B. Wasserstoffperoxid) und
- die Komponente (A3) mindestens ein zweites Oxidationsmittel (z.B. ein oder mehrere Poroxodisulfatsalze) enthält.
mindestens eine der Komponenten (A1), (A2) und (A3) enthält hierbei wieder mindestens ein nichtionisches Tensid (b).

Während der Einwirkzeit der Mittel auf der Faser kann es vorteilhaft sein, den Aufhellvorgang oder Mattiervorgang durch Wärmezufuhr zu unterstützen. Die Wärmezufuhr kann durch eine externe Wärmequelle, wie z.B. warme Luft eines Warmluftgebläses, als auch, insbesondere bei einer Haaraufhellung am lebenden Probanden, durch die Körpertemperatur des Probanden erfolgen. Bei letzterer Möglichkeit wird üblicherweise die behandelte Partie mit einer Haube abgedeckt. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Nach Ende der Einwirkzeit wird die verbleibende Färbezubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Wasser erfolgen kann, wenn das Aufhellmittel einen stark tensidhaltigen Träger besitzt.

Bezüglich weiterer bevorzugter Ausführungsformen der erfindungsgemäßen Verwendungen gilt mutatis mutandis das zu den erfindungsgemäßen Mitteln Gesagte.

### Beispiele

### Direkzieher 1: 3-Methyl-2-(2-{4-[methyl({3-[methyl({4-[2-(3-methyl-1,3-thiazol-3-ium-2-yl)diazen-1-yl]phenyl})amino]propyl})amino]phenyl}diazen-1-yl)-1,3-thiazol-3-ium, Di(methylsulfat)

### (DZ 1, erfindungsgemäß)

Der Farbstoff DZ 1 wurde nach einem Verfahren synthetisiert, wie es in den Dokumenten WO 2002/100369 A2 und US 3291788 beschrieben ist.

Als Edukte wurden 2-Aminothiazol und N,N'-Dimethyl-N,N'-diphenyl-propan-1,3-diamin eingesetzt (Azokupplungsreaktion in wässriger, schwefelsauerer Lösung mit Nitrosylschwefelsäure). Der in dieser Azokupplungsreaktion entstehende neutrale Farbstoff wurde im Anschluss daran quaterniert (beispielsweise mit dem Quaternierungsmittel Dimethylsulfat in einem polaren, aprotischen Lösungsmittel wie Dimethylformamid oder Dimethylsulfoxid).

### DZ 1 (erfindungsgemäß)

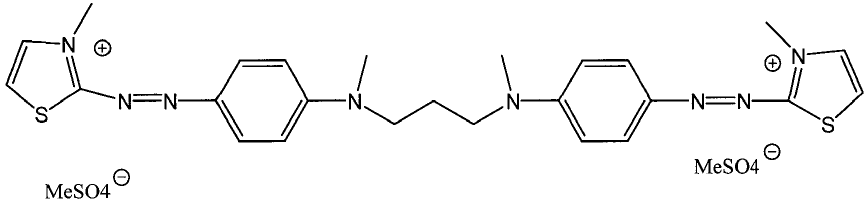

### Vergleichsbeispiel

Direkzieher 2: 3-Methyl-2-(2-{4-[methyl({2-[methyl({4-[2-(3-methyl-1,3-thiazol-3-ium-2-yl)diazen-1-yl]phenyl})amino]ethyl})amino]phenyl}diazen-1-yl)-1,3-thiazol-3-ium Di(methylsulfat) (DZ 2, Vergleich) Der Farbstoff DZ 2 wurde nach einem Verfahren synthetisiert, wie es in den Dokumenten WO 2002/100369 A2 und US 3291788 beschrieben ist.

Als Edukte wurden 2-Aminothiazol und N,N'-Dimethyl-N,N'-diphenyl-ethan-1,2-diamin eingesetzt (Azokupplungsreaktion in wässriger, schwefelsauerer Lösung mit Nitrosylschwefelsäure). Der in dieser Azokupplungsreaktion entstehende Farbstoff wurde im Anschluss daran quaterniert (beispielsweise mit einem Quaternierungsmittel wie Dimethylsulfat in einem polaren, aprotischen Lösungsmittel wie Dimethylformamid oder Dimethylsulfoxid).

### DZ 2 (Vergleich):

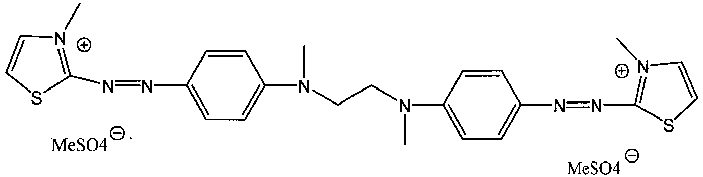

### Färbebeispiele

### Formulierungen

Es wurden die folgenden Färbecremes hergestellt (alle Angaben in Gew.-%, Aktivsubstanz)

| | **V1** | **V2** | **V3** | **E1** |
|---|---|---|---|---|
| Cetearyl Alcohol (C16/C18-Fettalkohol) | 1,0 | 1,0 | 1,0 | 1,0 |
| Coconut Alcohol (C12/C18-Fettalkohol) | 1,0 | 1,0 | 1,0 | 1,0 |
| Methylparaben | 0,1 | 0,1 | 0,1 | 0,1 |
| Propylparaben | 0,1 | 0,1 | 0,1 | 0,1 |
| Cocoamidopropylbetain (zwitterionisches Tensid) | --- | 3,0 | 3,0 | --- |
| Ceteareth-12 (nichtionisches Tensid) | 1,5 | --- | --- | 1,5 |
| Ceteareth-20 (nichtionisches Tensid) | 1,5 | --- | --- | 1,5 |
| DZ 1 (erfindungsgemäß) | --- | --- | 1,0 | 1,0 |
| DZ 2 (Vergleich) | 1,0 | 1,0 | --- | --- |
| Ammoniumsulfat | 1,0 | 1,0 | 1,0 | 1,0 |
| Wasser | ad 100 | ad 100 | ad 100 | ad 100 |

Die Fettalkohole (Fettstoffe) wurden zusammen mit den Parabenen aufgeschmolzen. Diese Schmelze wurde mit den jeweils eingesetzten Tensiden und heißem Wasser emulgiert, anschließend wurde der in Propylenglycol vorgelöste Farbstoff hinzu gegeben sowie die Ammoniumsulfatlösung hinzugefügt. Der angegebene pH-Wert wurde mit Ammoniak eingestellt, danach wurde mit Wasser auf 100 g aufgefüllt.

### Ausfärbungen

Jeweils 1,8 g der Färbecreme wurden auf eine ca. 6 cm lange Strähne Menschenhaares (Kerling Euronaturhaar,80 % ergraut) aufgetragen und dort 30 Minuten bei 30 °C belassen. Nach Beendigung der Einwirkzeit wurde das Haar ausgespült, mit einem üblichen Haarwaschmittel gewaschen und anschließend getrocknet. Nach dem Trocknen wurden die Färbung und die Farbintensität der Strähnen visuell unter der Tageslichtlampe beurteilt.

| | Formulierung | pH-Wert | Farbnuance | Farbintensität |
|---|---|---|---|---|
| V1 | DZ 2 (Vergleich) mit nichtionischen Tensiden | 9,5 | grau (Ausgangshaarfarbe, kein Farbaufzug) | + |
| V2 | DZ 2 (Vergleich) mit zwitterionischem Tensid | 9,5 | grau (Ausgangshaarfarbe, kein Farbaufzug) | + |
| V3 | DZ 1 (erfindungsgemäßer DZ) mit zwitterionischem Tensid | 9,5 | dunkelviolett | ++++ |
| E1 | DZ 1 (erfindungsgemäßer DZ) mit nichtionischem Tensid | 9,5 | schwarzblau | +++++ |

| | | | | |
|---|---|---|---|---|
| Farbintensität: + = schlecht +++ = mittel +++++ = sehr gut | | | | |

Bei den Formulierungen V1 und V2 handelt es sich um Vergleichsformulierungen, welche den nicht erfindungsgemäßen direktziehenden Farbstoff DZ 2 enthielten (DZ 2: 3-Methyl-2-(2-{4-[methyl({2-[methyl({4-[2-(3-methyl-1,3-thiazol-3-ium-2-yl)diazen-1-yl]phenyl})amino]ethyl})amino]phenyl}diazen-1-yl)-1,3-thiazol-3-ium Di(methylsulfat) (Vergleich)).

Bei Ausfärbung von V1 und V2 konnte kein Farbaufzug beobachtet werden, die Strähnen waren wie die Ausgangshaarfarbe (Kerling, zu 80 % ergraut) grau gefärbt.

Bei der Formulierung V3 handelt es sich um eine Vergleichsformulierung, welche zwar einen direktziehenden Farbstoff der allgemeinen Formel (I), jedoch anstatt eines nichtionischen Tensids das zwitterionische Tensid Cocoamidopropylbetain (Alternativname: {[3-(Dodecanoylamino)propyl]-(dimethyl)ammonio}acetat), CAS-Nr. 61789-40-0) enthielt. Die mit dieser Formulierung wurde die Haarsträhne dunkelviolett gefärbt.

Bei der Formulierung E1 handelte es sich um eine erfindungsgemäße Formulierung, welche den erfindungsgemäßen direktziehenden Farbstoff DZ1 in Kombination mit nichtionischen Tensiden enthielt. (DZ1 = 3-Methyl-2-(2-{4-[methyl({3-[methyl({4-[2-(3-methyl-1,3-thiazol-3-ium-2-yl)diazen-1-yl]phenyl})-amino]propyl})amino]phenyl}diazen-1-yl)-1,3-thiazol-3-ium, Di(methylsulfat)). Es wurde eine intensive, tief dunkelblaue, fast schwarze Färbung ohne Rotanteil erhalten.

Mit der Formulierung E1 konnte das intensivste Farbergebnis erzielt werden.

## Patentansprüche

1. Mittel zum Färben von keratinischen Fasern, insbesondere menschlichen Haaren, enthaltend in einem kosmetischen Träger
(a) mindestens einen direktziehenden Farbstoff der Formel (I), 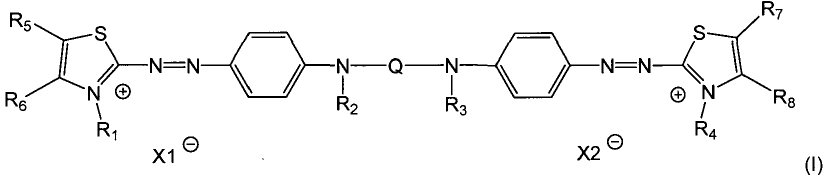 wobei
R1, R4 unabhängig voneinander für eine C₁-C₆-Alkylgruppe stehen,
R2, R3 unabhängig voneinander für ein Wasserstoffatom oder eine C₁-C₆-Alkylgruppe, stehen,
R5, R6, R7, R8 jeweils für ein Wasserstoffatom stehen,
X1, X2 unabhängig voneinander für ein physiologisch verträgliches Anion, bevorzugt aus der Gruppe Chlorid, Bromid, lodid, Methylsulfat, Methylsulfonat, p-Toluolsulfonat, Acetat, Hydrogensulfat, ½ Sulfat oder ½ Tetrachlorozinkat, stehen,
Q für eine Gruppierung der Formel (II) steht,
*-(CH₂)n-* (II)
n für eine ganze Zahl von 3 bis 6 steht,
und
(b) mindestens ein nichtionisches Tensid.

2. Mittel nach Anspruch 1, **dadurch gekennzeichnet, dass** es (a) mindestens einen direktziehenden Farbstoff der allgemeinen Formel (I) enthält, der ausgewählt ist aus
- Salzen des 3-Methyl-2-[2-(4-{[3-({4-[2-(3-methyl-1,3-thiazol-3-ium-2-yl)diazen-1-yl]phenyl}amino)propyl]amino}phenyl)diazen-1-yl]-1,3-thiazol-3-iums
- Salzen des 3-Methyl-2-[2-(4-{[4-({4-[2-(3-methyl-1,3-thiazol-3-ium-2-yl)diazen-1-yl]phenyl}amino)butyl]amino}phenyl)diazen-1-yl]-1,3-thiazol-3-iums
- Salzen des 3-Methyl-2-[2-(4-{[5-({4-[2-(3-methyl-1,3-thiazol-3-ium-2-yl)diazen-1-yl]phenyl}amino)pentyl]amino}phenyl)diazen-1-yl]-1,3-thiazol-3-iums
- Salzen des 3-Methyl-2-(2-{4-[methyl({3-[methyl({4-[2-(3-methyl-1,3-thiazol-3-ium-2-yl)diazen-1-yl]phenyl})amino]propyl})amino]phenyl}diazen-1-yl)-1,3-thiazol-3-iums
- Salzen des 3-Methyl-2-(2-{4-[methyl({4-[methyl({4-[2-(3-methyl-1,3-thiazol-3-ium-2-yl)diazen-1-yl]phenyl})amino]butyl})amino]phenyl}diazen-1-yl)-1,3-thiazol-3-iums
- Salzen des 3-Methyl-2-(2-{4-[methyl({5-[methyl({4-[2-(3-methyl-1,3-thiazol-3-ium-2-yl)diazen-1-yl]phenyl})amino]pentyl})amino]phenyl}diazen-1-yl)-1,3-thiazol-3-iums
- Salzen des 2-(2-{4-[Ethyl({3-[ethyl({4-[2-(3-methyl-1,3-thiazol-3-ium-2-yl)diazen-1-yl]phenyl})amino]propyl})amino]phenyl}diazen-1-yl)-3-methyl-1,3-thiazol-3-iums
- Salzen des 2-(2-{4-[Ethyl({4-[ethyl({4-[2-(3-methyl-1,3-thiazol-3-ium-2-yl)diazen-1-yl]phenyl})amino]butyl})amino]phenyl}diazen-1-yl)-3-methyl-1,3-thiazol-3-iums
- Salzen des 2-(2-{4-[Ethyl({5-[ethyl({4-[2-(3-methyl-1,3-thiazol-3-ium-2-yl)diazen-1-yl]phenyl})amino]pentyl})amino]phenyl}diazen-1-yl)-3-methyl-1,3-thiazol-3-iums
- Salzen des 3-Ethyl-2-[2-(4-{[3-({4-[2-(3-ethyl-1,3-thiazol-3-ium-2-yl)diazen-1-yl]phenyl}amino)propyl]amino}phenyl)diazen-1-yl]-1,3-thiazol-3-iums
- Salzen des 3-Ethyl-2-[2-(4-{[4-({4-[2-(3-ethyl-1,3-thiazol-3-ium-2-yl)diazen-1-yl]phenyl}amino)butyl]amino}phenyl)diazen-1-yl]-1,3-thiazol-3-iums
- Salzen des 3-Ethyl-2-[2-(4-{[5-({4-[2-(3-ethyl-1,3-thiazol-3-ium-2-yl)diazen-1-yl]phenyl}amino)pentyl]amino}phenyl)diazen-1-yl]-1,3-thiazol-3-iums
- Salzen des 3-Ethyl-2-[2-(4-{[3-({4-[2-(3-ethyl-1,3-thiazol-3-ium-2-yl)diazen-1-yl]phenyl}(methyl)amino)propyl](methyl)amino}phenyl)diazen-1-yl]-1,3-thiazol-3-iums
- Salzen des 3-ethyl-2-[2-(4-{[4-({4-[2-(3-ethyl-1,3-thiazol-3-ium-2-yl)diazen-1-yl]phenyl}(methyl)amino)butyl](methyl)amino}phenyl)diazen-1-yl]-1,3-thiazol-3-iums
- Salzen des 3-Ethyl-2-[2-(4-{[5-({4-[2-(3-ethyl-1,3-thiazol-3-ium-2-yl)diazen-1-yl]phenyl}(methyl)amino)pentyl](methyl)amino}phenyl)diazen-1-yl]-1,3-thiazol-3-iums
- Salzen des 3-Ethyl-2-(2-{4-[ethyl({3-[ethyl({4-[2-(3-ethyl-1,3-thiazol-3-ium-2-yl)diazen-1-yl]phenyl})amino]propyl})amino]phenyl}diazen-1-yl)-1,3-thiazol-3
- Salzen des 3-Ethyl-2-(2-{4-[ethyl({4-[ethyl({4-[2-(3-ethyl-1,3-thiazol-3-ium-2-yl)diazen-1-yl]phenyl})amino]butyl})amino]phenyl}diazen-1-yl)-1,3-thiazol-3-iums und/oder
- Salzen des 3-Ethyl-2-(2-{4-[ethyl({5-[ethyl({4-[2-(3-ethyl-1,3-thiazol-3-ium-2-yl)diazen-1-yl]phenyl})amino]pentyl})amino]phenyl}diazen-1-yl)-1,3-thiazol-3-iums

3. Mittel nach einem der Ansprüche 1 bis 2, **dadurch gekennzeichnet, dass** es - bezogen auf das Gesamtgewicht des Mittels- einen oder mehrere direktziehende Farbstoffe (a) der Formel (I) in einer Gesamtmenge von 0,01 bis 4,5 Gew.-%, bevorzugt von 0,05 bis 2,8 Gew.-%, weiter bevorzugt von 0,1 bis 2,2 Gew.-% und besonders bevorzugt von 0,2 bis 1,2 Gew.-% enthält.

4. Mittel nach einem der Ansprüche 1 bis 3, **dadurch gekennzeichnet, dass** es (b) mindestens ein nichtionisches Tensid enthält, das ausgewählt ist aus den
- Anlagerungsprodukten von 2 bis 100 Mol Ethylenoxid und/oder 2 bis 100 Mol Propylenoxid an lineare und verzweigte Fettalkohole mit 12 bis 30 C-Atomen, die Fettalkoholpolyglykolether bzw. die Fettalkoholpolypropylenglycolether bzw. gemischte Fettalkoholpolyether,
- Anlagerungsprodukten von 2 bis 100 Mol Ethylenoxid und/oder 2 bis 100 Mol Propylenoxid an lineare und verzweigte Fettsäuren mit 12 bis 30 C-Atomen, die Fettsäurepolyglykolether bzw. die Fettsäurepolypropylenglykolether bzw. gemischte Fettsäurepolyether, und der
- Anlagerungsprodukten von 5 bis 60 Mol Ethylenoxid an Rizinusöl und hydriertes Rizinusöl.

5. Mittel nach einem der Ansprüche 1 bis 4, **dadurch gekennzeichnet, dass** es als nichtionisches Tensid (b) mindestens eine Verbindung der Formel (B1) und/oder mindestens eine Verbindung der Formel (B2) enthält, 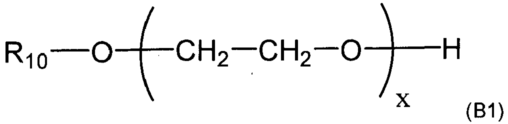 wobei
R10 für eine lineare oder verzweigte C₁₂-C₃₀-Alkylgruppe steht und
x für eine ganze Zahl von 2 bis 100 steht, 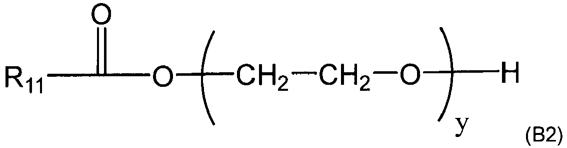
wobei
R11 für eine lineare oder verzweigte C₁₁-C₂₉-Alkylgruppe steht und
y für eine ganze Zahl von 2 bis 100 steht.

6. Mittel nach einem der Ansprüche 1 bis 5, **dadurch gekennzeichnet, dass** es als nichtionisches Tensid (b) mindestens eine Verbindung der Formel (B1) enthält, 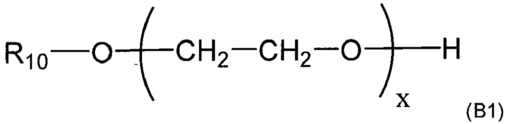 wobei
R10 für eine lineare oder verzweigte C₁₂-C₃₀-Alkylgruppe steht und
x für eine ganze Zahl von 10 bis 50 steht.

7. Mittel nach einem der Ansprüche 1 bis 6, **dadurch gekennzeichnet, dass** es - bezogen auf das Gesamtgewicht des Mittels - ein oder mehrere nichtionische Tenside (b) in einer Gesamtmenge von 0,2 bis 10,5 Gew.-%, bevorzugt von 1,0 bis 7,5 Gew.-%, weiter bevorzugt von 2,0 bis 4,5 Gew.-% und ganz besonders bevorzugt von 2,5 bis 4,5 Gew.-% enthält.

8. Mittel nach einem der Ansprüche 1 bis 7, **dadurch gekennzeichnet, dass** es zusätzlich mindestens einen weiteren kationischen direktziehenden Farbstoff enthält, der von den Farbstoffen der Formel (I) verschieden ist.

9. Mittel nach einem der Ansprüche 1 bis 8, **dadurch gekennzeichnet, dass** es zusätzlich Basic Orange 31 und/oder Basic Red 51 enthält.

10. Mittel nach einem der Ansprüche 1 bis 9, **dadurch gekennzeichnet, dass** es - bezogen auf das Gesamtgewicht des Mittels - 0,5 bis 12,5 Gew.-%, bevorzugt 2,5 bis 10 Gew.-% und insbesondere 3 bis 6 Gew.-% Wasserstoffperoxid enthält.

11. Mittel nach einem der Ansprüche 1 bis 10, **dadurch gekennzeichnet, dass** es zusätzlich mindestens ein Persulfat aus der Gruppe Ammoniumperoxodisulfat, Kaliumperoxodisulfat und Natriumperoxodisulfat enthält.

## Claims

1. An agent for dyeing keratin fibers, in particular human hair, containing, in a cosmetic carrier,
(a) at least one direct dye of formula (I), 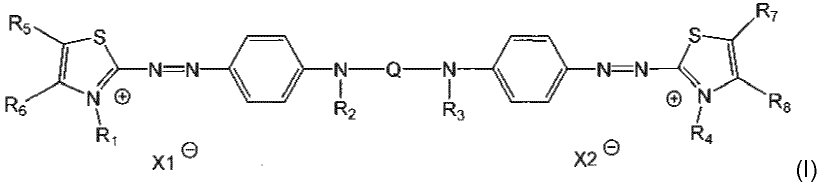 in which
R1, R4 represent, independently of one another, a C₁-C₆ alkyl group,
R2, R3 represent, independently of one another, a hydrogen atom or a C₁-C₆ alkyl group,
R5, R6, R7, R8 each represent a hydrogen atom,
X1, X2 represent, independently of one another, a physiologically acceptable anion, preferably from the group of chloride, bromide, iodide, methyl sulfate, methyl sulfonate, p-toluene sulfonate, acetate, hydrogen sulfate, ½ sulfate or ½ tetrachlorozincate,
Q represents a group of formula (II),
*-(CH₂)n-* (II)
n represents an integer from 3 to 6,
and
(b) at least one non-ionic surfactant.

2. The agent according to claim 1, **characterized in that** it contains (a) at least one direct dye of general formula (I), which dye is selected from
- salts of 3-methyl-2-[2-(4-{[3-({4-[2-(3-methyl-1,3-thiazol-3-ium-2-yl)-diazen-1-yl]phenyl}amino)propyl]amino}phenyl)diazen-1-yl]-1,3-thiazol-3-ium
- salts of 3-méthyl-2-[2-(4-{[4-({4-[2-(3-méthyl-1,3-thiazol-3-ium-2-yl)-diazen-1-yl]phenyl}amino)butyl]amino}phenyl)diazen-1-yl]-1,3-thiazol-3-ium
- salts of 3-methyl-2-[2-(4-{[5-({4-[2-(3-methyl-1,3-thiazol-3-ium-2-yl)diazen-1-yl]phenyl}amino)pentyl]amino}phenyl)diazen-1-yl]-1,3-thiazol-3-ium
- salts of 3-methyl-2-(2-{4-[methyl({3-[methyl({4-[2-(3-methyl-1,3-thiazol-3-ium-2-yl)diazen-1-yl]phenyl})amino]propyl})amino]phenyl}diazen-1-yl)-1,3-thiazol-3-ium
- salts of 3-methyl-2-(2-{4-[methyl({4-[methyl({4-[2-(3-methyl-1,3-thiazol-3-ium-2-yl)diazen-1-yl]phenyl})amino]butyl})amino]phenyl}diazen-1-yl)-1,3-thiazol-3-ium
- salts of 3-methyl-2-(2-{4-[methyl({5-[methyl({4-[2-(3-methyl-1,3-thiazol-3-ium-2-yl)diazen-1-yl]phenyl})amino]pentyl})amino]phenyl}diazen-1-yl)-1,3-thiazol-3-ium
- salts of 2-(2-{4-[ethyl({3-[ethyl({4-[2-(3-methyl-1,3-thiazol-3-ium-2-yl)diazen-1-yl]phenyl})amino]propyl})amino]phenyl}diazen-1-yl)-3-methyl-1,3-thiazol-3-ium
- salts of 2-(2-{4-[éthyl({4-[éthyl({4-[2-(3-methyl-1,3-thiazol-3-ium-2-yl)diazen-1-yl]phenyl})amino]butyl})amino]phenyl}diazen-1-yl)-3-methyl-1,3-thiazol-3-ium
- salts of 2-(2-{4-[ethyl({5-[ethyl({4-[2-(3-methyl-1,3-thiazol-3-ium-2-yl)diazen-1-yl]phenyl})amino]pentyl})amino]phenyl}diazen-1-yl)-3-methyl-1,3-thiazol-3-ium
- salts of 3-ethyl-2-[2-(4-{[3-({4-[2-(3-ethyl-1,3-thiazol-3-ium-2-yl)diazen-1-yl]phenyl}amino)propyl]amino}phenyl)diazen-1-yl]-1,3-thiazol-3-ium
- salts of 3-ethyl-2-[2-(4-{[4-({4-[2-(3-ethyl-1,3-thiazol-3-ium-2-yl)diazen-1-yl]phenyl}amino)butyl]amino}phenyl)diazen-1-yl]-1,3-thiazol-3-ium
- salts of 3-ethyl-2-[2-(4-{[5-({4-[2-(3-ethyl-1,3-thiazol-3-ium-2-yl)diazen-1-yl]phenyl}amino)pentyl]amino}phenyl)diazen-1-yl]-1,3-thiazol-3-ium
- salts of 3-ethyl-2-[2-(4-{[3-({4-[2-(3-ethyl-1,3-thiazol-3-ium-2-yl)diazen-1-yl]phenyl}(methyl)amino)propyl](methyl)amino}phenyl)diazen-1-yl]-1,3-thiazol-3-ium
- salts of 3-ethyl-2-[2-(4-{[4-({4-[2-(3-ethyl-1,3-thiazol-3-ium-2-yl)diazen-1-yl]phenyl}(methyl)amino)butyl](methyl)amino}phenyl)diazen-1-yl]-1,3-thiazol-3-ium
- salts of 3-ethyl-2-[2-(4-{[5-({4-[2-(3-ethyl-1,3-thiazol-3-ium-2-yl)diazen-1-yl]phenyl}(methyl)amino)pentyl](methyl)amino}phenyl)diazen-1-yl]-1,3-thiazol-3-ium
- salts of 3-ethyl-2-(2-{4-[ethyl({3-[ethyl({4-[2-(3-ethyl-1,3-thiazol-3-ium-2-yl)diazen-1-yl]phenyl})amino]propyl})amino]phenyl}diazen-1-yl)-1,3-thiazol-3
- salts of 3-ethyl-2-(2-{4-[ethyl({4-[ethyl({4-[2-(3-ethyl-1,3-thiazol-3-ium-2-yl)diazen-1-yl]phenyl})amino]butyl})amino]phenyl}diazen-1-yl)-1,3-thiazol-3-ium and/or
- salts of 3-ethyl-2-(2-{4-[ethyl({5-[ethyl({4-[2-(3-ethyl-1,3-thiazol-3-ium-2-yl)diazen-1-yl]phenyl})amino]pentyl})amino]phenyl}diazen-1-yl)-1,3-thiazol-3-ium.

3. The agent according to one of claims 1 to 2, **characterized in that** it contains, based on the total weight of the agent, one or more direct dyes (a) of formula (I) in a total amount of from 0.01 to 4.5 wt.%, preferably from 0.05 to 2.8 wt.%, more preferably from 0.1 to 2.2 wt.%, and particularly preferably from 0.2 to 1.2 wt.%.

4. The agent according to one of claims 1 to 3, **characterized in that** it contains (b) at least one non-ionic surfactant selected from
- addition products of 2 to 100 mol ethylene oxide and/or 2 to 100 mol propylene oxide to linear and branched fatty alcohols having 12 to 30 C atoms, the fatty alcohol polyglycol ethers or the fatty alcohol polypropylene glycol ethers or mixed fatty alcohol polyethers,
- addition products of 2 to 100 mol ethylene oxide and/or 2 to 100 mol propylene oxide to linear and branched fatty alcohols having 12 to 30 C atoms, the fatty acid polyglycol ethers or the fatty acid polypropylene glycol ethers or mixed fatty alcohol polyethers, and
- addition products of 5 to 60 mol ethylene oxide to castor oil and hydrogenated castor oil.

5. The agent according to one of claims 1 to 4, **characterized in that** it contains, as non-ionic surfactant (b), at least one compound of formula (B1) and/or at least one compound of formula (B2), 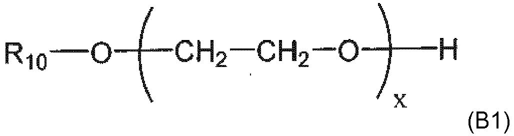 in which
R10 represents a linear or branched C₁₂-C₃₀ alkyl group and
x represents an integer from 2 to 100, 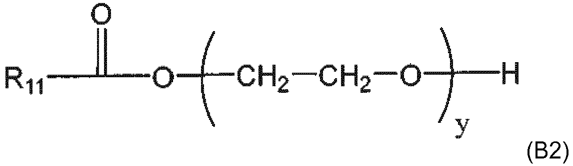
in which
R11 represents a linear or branched C₁₁-C₂₉ alkyl group and
y represents an integer from 2 to 100.

6. The agent according to one of claims 1 to 5, **characterized in that** it contains, as non-ionic surfactant (b), at least one compound of formula (B1), 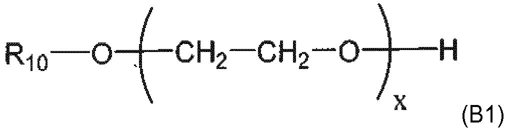 in which
R10 represents a linear or branched C₁₂-C₃₀ alkyl group and
x represents an integer from 10 to 50.

7. The agent according to one of claims 1 to 6, **characterized in that** it contains, based on the total weight of the agent, one or more non-ionic surfactants (b) in a total amount of from 0.2 to 10.5 wt.%, preferably from 1.0 to 7.5 wt.%, more preferably from 2.0 to 4.5 wt.%, and very particularly preferably from 2.5 to 4.5 wt.%.

8. The agent according to one of claims 1 to 7, **characterized in that** it additionally contains at least one additional cationic direct dye that is different from the dyes of formula (I).

9. The agent according to one of claims 1 to 8, **characterized in that** it additionally contains Basic Orange 31 and/or Basic Red 51.

10. The agent according to one of claims 1 to 9, **characterized in that** it contains, based on the total weight of the agent, 0.5 to 12.5 wt.%, preferably 2.5 to 10 wt.%, and in particular 3 to 6 wt.%, hydrogen peroxide.

11. The agent according to one of claims 1 to 10, **characterized in that** it additionally contains at least one persulfate from the group of ammonium peroxodisulfate, potassium peroxodisulfate and sodium peroxodisulfate.

## Revendications

1. Composition pour la coloration des fibres kératiniques, en particulier de cheveux humains, contenant dans un support cosmétique
(a) au moins un colorant direct de formule (I), 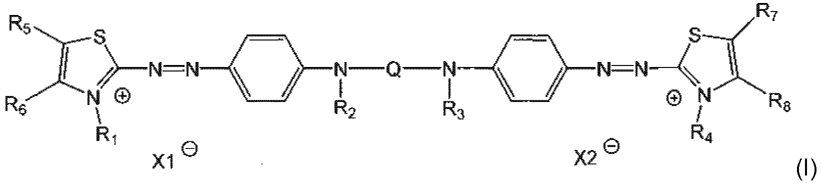 dans laquelle
R1, R4 représentent chacun indépendamment l'un de l'autre un groupe alkyle C₁-C₆,
R2, R3 représentent chacun indépendamment l'un de l'autre un atome d'hydrogène ou un groupe alkyle C₁-C₆,
R5, R6, R7, R8 représentent chacun un atome d'hydrogène,
X1, X2 représentent chacun indépendamment l'un de l'autre un anion physiologiquement acceptable, de préférence choisi dans le groupe constitué par le chlorure, le bromure, l'iodure, le sulfate de méthyle, le méthylsulfonate, le p-toluènesulfonate, l'acétate, l'hydrogénosulfate, ½ sulfate ou ½ tétrachlorozincate,
Q représente un groupement de formule (II), *-(CH₂)n-* (II)
n représente un nombre entier allant de 3 à 6,
et
(b) au moins un agent tensioactif non ionique

2. Composition selon la revendication 1, **caractérisée en ce qu'**elle (a) contient au moins un colorant direct de formule générale (I) qui est choisi parmi
- les sels du 3-méthyl-2-[2-(4-{[3-({4-[2-(3-méthyl-1,3-thiazole-3-ium-2-yl)diazen-1-yl]phényl}amino)propyl]amino}phényl)diazen-1-yl]-1,3-thiazole-3-iums
- les sels du 3-méthyl-2-[2-(4-{[4-({4-[2-(3-méthyl-1,3-thiazole-3-ium-2-yl)diazen-1-yl]phényl}amino)butyl]amino}phényl)diazen-1-yl]-1,3-thiazole-3-iums
- les sels du 3-méthyl-2-[2-(4-{[5-({4-[2-(3-méthyl-1,3-thiazole-3-ium-2-yl)diazen-1-yl]phényl}amino)pentyle]amino}phényl)diazen-1-yl]-1,3-thiazole-3-iums
- les sels du 3-méthyl-2-(2-{4-[méthyl({3-[méthyl({4-[2-(3-méthyl-1,3-thiazole-3-ium-2-yl)diazen-1-yl]phényl})amino]propyl})amino]phényl}diazen-1-yl)-1,3-thiazole-3-iums
- les sels du 3-méthyl-2-(2-{4-[méthyl({4-[méthyl({4-[2-(3-méthyl-1,3-thiazole-3-ium-2-yl)diazen-1-yl]phényl})amino]butyl})amino]phényl}diazen-1-yl)-1,3-thiazole-3-iums
- les sels du 3-méthyl-2-(2-{4-[méthyl({5-[méthyl({4-[2-(3-méthyl-1,3-thiazole-3-ium-2-yl)diazen-1-yl]phényl})amino]pentyl})amino]phényl}diazen-1-yl)-1,3-thiazole-3-iums
- les sels de 2-(2-{4-[éthyl({3-[éthyl({4-[2-(3-méthyl-1,3-thiazole-3-ium-2-yl)diazen-1-yl]phényl})amino]propyl})amino]phényl}diazen-1-yl)-3-méthyl-1,-thiazole-3-iums
- les sels de 2-(2-{4-[éthyl({4-[éthyl({4-[2-(3-méthyl-1,3-thiazole-3-ium-2-yl)diazen-1-yl]phényl})amino]butyl})amino]phényl}diazen-1-yl)-3-méthyl-1,3-thiazole-3-iums
- les sels de 2-(2-{4-[éthyl({5-[éthyl({4-[2-(3-méthyl-1,3-thiazole-3-ium-2-yl)diazen-1-yl]phényl})amino]pentyl})amino]phényl}diazén-1-yl)-3-méthyl-1,3-thiazole-3-iums
- les sels de 3-éthyl-2-[2-(4-{[3-({4-[2-(3-éthyl-1,3-thiazole-3-ium-2-yl)diazen-1-yl]phényl}amino)propyl]amino}phényl)diazen-1-yl]-1,3-thiazole-3-iums
- les sels de 3-éthyl-2-[2-(4-{[4-({4-[2-(3-éthyl-1,3-thiazole-3-ium-2-yl)diazen-1-yl]phényl}amino)butyl]amino}phényl)diazen-1-yl]-1,3-thiazole-3-iums
- les sels de 3-éthyl-2-[2-(4-{[5-({4-[2-(3-éthyl-1,3-thiazole-3-ium-2-yl)diazen-1-yl]phényl}amino)pentyl]amino}phényl)diazen-1-yl]-1,3-thiazole-3-iums
- les sels de 3-éthyl-2-[2-(4-{[3-({4-[2-(3-éthyl-1,3-thiazole-3-ium-2-yl)diazen-1-yl]phényl}(méthyl)amino)propyl](méthyl)amino}phényl)diazen-1-yl]-1,3-thiazole-3-iums
- les sels de 3-éthyl-2-[2-(4-{[4-({4-[2-(3-éthyl-1,3-thiazole-3-ium-2-yl)diazen-1-yl]phényl}(méthyl)amino)butyl](méthyl)amino}phényl)diazen-1-yl]-1,3-thiazole-3-iums
- les sels de 3-éthyl-2-[2-(4-{[5-({4-[2-(3-éthyl-1,3-thiazole-3-ium-2-yl)diazen-1-yl]phényl}(méthyl)amino)pentyl](méthyl)amino}phényl)diazen-1-yl]-1,3-thiazole-3-iums
- les sels de 3-éthyl-2-(2-{4-[éthyl({3-[éthyl({4-[2-(3-éthyl-1,3-thiazole-3-ium-2-yl)diazen-1-yl]phényl})amino]propyl})amino]phényl}diazén-1-yl)-1,3-thiazole-3
- les sels de 3-éthyl-2-(2-{4-[éthyl({4-[éthyl({4-[2-(3-éthyl-1,3-thiazole-3-ium-2-yl)diazen-1-yl]phényl})amino]butyl})amino]phényl}diazen-1-yl)-1,3-thiazole-3-iums et/ou
- les sels de 3-éthyl-2-(2-{4-[éthyl({5-[éthyl({4-[2-(3-éthyl-1,3-thiazole-3-ium-2-yl)diazen-1-yl]phényl})amino]pentyl})amino]phényl}diazen-1-yl)-1,3-thiazole-3-iums

3. Composition selon l'une des revendications 1 à 2, **caractérisée en ce qu'**elle contient un ou plusieurs colorants directs (a) de formule (I) en une quantité totale de 0,01 à 4,5 % en poids, de préférence de 0,05 à 2,8 % en poids, plus préférablement de 0,1 à 2,2 % en poids et de manière particulièrement préférée de 0,2 à 1,2 % en poids-par rapport au poids total de la composition-.

4. Composition selon l'une des revendications 1 à 3, **caractérisée en ce qu'**elle (b) contient au moins un agent tensioactif non ionique choisi dans le groupe constitué par
- des produits d'addition de 2 à 100 moles d'oxyde d'éthylène et/ou de 2 à 100 moles d'oxyde de propylène aux alcools gras linéaires et ramifiés ayant 12 à 30 atomes de carbone, les polyglycoléthers d'alcool gras ou les polypropylène glycoléthers d'alcool gras ou des polyéthers d'alcool gras mixtes,
- des produits d'addition de 2 à 100 moles d'oxyde d'éthylène et/ou de 2 à 100 moles d'oxyde de propylène aux acides gras linéaires et ramifiés ayant 12 à 30 atomes de carbone, les polyglycoléthers d'acides gras ou les polypropylèneglycoléthers d'acides gras ou polyéthers d'acides gras mixtes, et les
- produits d'addition de 5 à 60 moles d'oxyde d'éthylène à l'huile de ricin et à l'huile de ricin hydrogénée.

5. Composition selon l'une des revendications 1 à 4, **caractérisée en ce qu'**elle contient comme agent tensioactif non ionique (b) au moins un composé de formule (B1) et/ou au moins un composé de formule (B2), 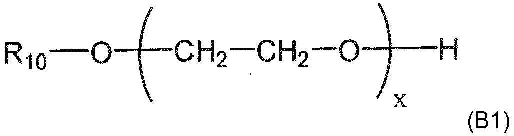 dans laquelle
R10 représente un groupe alkyle C₁₂-C₃₀-linéaire ou ramifié et
x représente un nombre entier allant de 2 à 100, 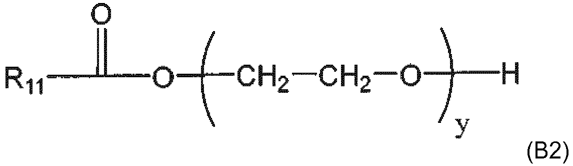
dans laquelle
R11 représente un groupe alkyle C₁₁-C₂₉-linéaire ou ramifié et
y représente un nombre entier allant de 2 à 100.

6. Composition selon l'une des revendications 1 à 5, **caractérisée en ce qu'**elle contient comme agent tensioactif non ionique (b) au moins un composé de formule (B1), 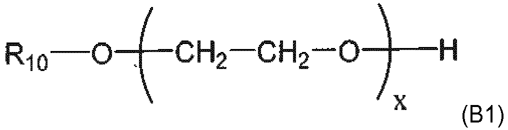 dans laquelle
R10 représente un groupe alkyle C₁₂-C₃₀-linéaire ou ramifié et
x représente un nombre entier allant de 10 à 50.

7. Composition selon l'une des revendications 1 à 6, **caractérisée en ce qu'**elle contient un ou plusieurs agents tensioactifs non ioniques (b) en une quantité totale de 0,2 à 10,5 % en poids, de préférence de 1,0 à 7,5 % en poids, plus préférablement de 2,0 à 4,5 % en poids et de manière tout particulièrement préférée de 2,5 à 4,5 % en poids-par rapport au poids total de la composition-.

8. Composition selon l'une des revendications 1 à 7, **caractérisée en ce qu'**elle contient en outre au moins un autre colorant direct cationique qui est différent des colorants de formule (I).

9. Composition selon l'une des revendications 1 à 8, **caractérisée en ce qu'**elle contient en outre Basic Orange 31 et/ou Basic Red 51.

10. Composition selon l'une des revendications 1 à 9, **caractérisée en ce qu'**elle contient 0,5 à 12,5 % en poids, de préférence 2,5 à 10 % en poids et en particulier 3 à 6 % en poids de peroxyde d'hydrogène- par rapport au poids total de la composition -.

11. Composition selon l'une des revendications 1 à 10, **caractérisée en ce qu'**elle contient en outre au moins un persulfate choisi dans le groupe constitué par le peroxodisulfate d'ammonium, le peroxodisulfate de potassium et le peroxodisulfate de sodium.
